# FASCICULE DE BREVET EUROPEEN

(11) **EP 0 820 731 B1**
(45) Date de publication et mention de la délivrance du brevet: **28.05.2003**
(21) Numéro de dépôt: 97401759.2
(22) Date de dépôt: 22.07.1997
(51) Int. Cl.: A61B 17/70

(54) **Implant squelettique**
Skelettimplantat
Skeletal implant

(30) Priorité: 22.07.1996 FR 9609157; 01.10.1996 FR 9611931
(43) Date de publication de la demande: 28.01.1998
(73) Titulaire: Zacouto, Fred, 75015 Paris (FR)
(72) Inventeur: Zacouto, Fred, 75015 Paris (FR)
(74) Mandataire: Jacobson, Claude

(56) Documents cités:
- EP-A- 0 385 929
- WO-A-94/06364
- FR-A- 2 723 841
- US-A- 3 648 294
- US-A- 4 932 969

## Description

La présente invention concerne un implant squelettique, et plus particulièrement un tel implant destiné à assurer une liaison entre au moins deux éléments du squelette, ledit implant étant réalisé en au moins deux parties, chacune étant susceptible d'être reliée à un desdits éléments.

Selon un premier aspect de l'invention, celle-ci concerne un implant de consolidation et/ou de fixation, et plus particulièrement un tel implant destiné à consolider une liaison entre deux éléments osseux, du type comprenant une première partie agencée pour être fixée à un des éléments et une deuxième partie agencée pour être fixée à l'autre élément.

On connaît de tels implants, susceptibles d'être utilisés par exemple dans le cas d'une réalisation de greffe osseuse ou lors de la formation d'un cal à la suite d'une fracture. Les deux extrémités de l'implant, rigidement liées l'une à l'autre par exemple parce qu'elles sont réalisées d'une seule pièce, sont fixées, par exemple vissées, chacune dans un élément osseux situé de part et d'autre de la greffe. Lorsque la greffe est consolidée, l'implant peut être retiré.

Toutefois, il est de nombreux cas où l'implant est laissé en place. Ceci est en particulier le cas lorsque l'implant est destiné à remplacer une structure osseuse impossible à reconstituer ou à constituer.

Dans de tels cas, la rigidité de l'implant, souvent indispensable au début de l'implantation, le temps de la formation du cal, constitue ultérieurement un inconvénient. En effet, les structures osseuses ne sont plus suffisamment sollicitées mécaniquement. Elles ne se reconstituent donc pas de façon optimale, cette reconstitution étant liée à une sollicitation satisfaisante de l'os dont le dérèglement a des conséquences qui peuvent entraîner des douleurs post-chirurgicales très difficiles à soigner.

En outre, lorsque l'implant est destiné à relier deux éléments osseux normalement susceptibles de se mouvoir l'un par rapport à l'autre, comme dans le cas d'un implant rachidien, cette rigidité entraîne un handicap fonctionnel chez le patient auquel il est adapté, et une sollicitation exagérée des articulations voisines.

Le document FR2723841 décrit un dispositif constituant une prothèse de disque intervertébral constitué de deux capsules déformables disposées dans l'espace intervertébral. Chaque capsule est constituée de deux plateaux rigides, parallèles en l'absence de contraintes extérieures, reliés par une enveloppe déformable de manière à constituer une enceinte étanche à volume variable, chaque enceinte étant remplie par un fluide à faible compressibilité.

La présente invention vise entre autres, à pallier ces inconvénients.

Selon un deuxième aspect de l'invention, celle-ci concerne un implant articulaire, et plus particulièrement un tel implant destiné soit à s'intercaler entre deux éléments osseux en mouvement relatif, tel qu'un disque intervertébral artificiel, soit à remplacer une articulation ou un élément d'une articulation, tel qu'une tête de fémur artificielle.

En ce qui concerne l'implant intercalaire, il peut être utile d'assister les structures osseuses adjacentes et les ligaments lors de mouvements rapides, ou même brutaux. En revanche, il peut être préférable de laisser ces structures et ligaments travailler lors de mouvements lents ou de simples charges statiques afin d'éviter leur atrophie ou leur affaiblissement.

En ce qui concerne l'implant articulaire proprement dit, une structure parfaitement rigide transmet intégralement à l'autre élément de l'articulation les chocs et les vibrations, entraînant un risque de dystrophie ou de rupture de cet autre élément.

La présente invention vise également à palier ces inconvénients.

A cet effet, l'invention a pour objet un implant squelettique, tel que décrit dans la revendication 1.

On observera en premier lieu que le réglage peut aussi bien être discret que continu et, par exemple, ne comporter que deux positions de réglage.

On sait qu'un dispositif amortisseur est un dispositif comportant d'une manière générale deux chambres de volumes variables remplies d'un fluide hydraulique et reliées par un orifice calibré. Un tel dispositif est destiné à "amortir" les mouvements entre deux organes dont l'un est relié à une structure de l'une des chambres et l'autre à un élément dans lequel est formé l'orifice calibré.

Lorsque les deux organes se déplacent l'un par rapport à l'autre, les volumes des deux chambres varient en sens inverse, le fluide hydraulique étant laminé au niveau de l'orifice calibré. Il en résulte une force s'opposant au mouvement relatif entre les deux organes, dont on montre qu'elle est proportionnelle à la vitesse de ce mouvement.

Utilisé dans le cadre de l'invention, un tel dispositif appliqué à un implant de type implant de consolidation et/ou de fixation présente l'avantage qu'il permet aux structures osseuses de fonctionner, et donc de se développer, de façon pratiquement normale aux vitesses modérées relatives entre les éléments osseux reliés par l'implant, et en particulier dans le cas de contraintes statiques. En revanche, plus les vitesses relatives sont importantes, notamment en cas de mouvement volontaire rapide ou de choc, et donc de contraintes dynamiques, plus la part de l'effort repris par l'implant est importante.

Il en résulte que la structure ostéo-ligamenteuse fragilisée par la situation ayant justifié la pose de l'implant, peut néanmoins fonctionner, et donc se reconstituer normalement tant que les sollicitations restent modérées. Mais plus ces sollicitations sont importantes, plus la structure naturelle est assistée par l'implant.

Par ailleurs, le coefficient d'amortissement étant réglable, il est possible de le diminuer progressivement au fur et à mesure que la structure osseuse se reconstitue. Cette dernière peut ainsi prendre en charge de plus en plus de sollicitations dynamiques jusqu'à, éventuellement, retrouver un fonctionnement normal.

On observera qu'il avait déjà été suggéré dans l'art antérieur de doter des prothèses de moyens visqueux et/ou élastiques destinés à amortir les chocs. De même, il est bien connu de prévoir, sur certaines prothèses, des moyens de réglage ou d'adaptation. On n'avait toutefois pas encore proposé de prothèse à caractéristique de fonctionnement d'amortissement visqueux, dans laquelle le coefficient d'amortissement serait réglable. Cette combinaison est essentielle dans la fonction première de l'invention, qui est de permettre une reconstruction progressive de la structure osseuse et une adaptation optimale permanente à l'état de cette structure.

Appliquée à un implant articulaire, l'invention permet en outre de donner plus de souplesse à l'articulation, ce qui lui permet, comme dans l'art antérieur, d'amortir les chocs, mais ici en laissant travailler les structures osseuses avoisinantes et les ligaments.

Enfin, cette caractéristique d'amortissement des chocs a pour conséquence de protéger des chocs l'implant lui-même ainsi que les articulations se trouvant au dessus et au dessous de l'implant.

Dans un mode de réalisation particulier, l'implant comprend des moyens amovibles de blocage du dispositif amortisseur à une longueur prédéterminée.

L'implant selon l'invention peut donc dans ce mode de réalisation fonctionner dans un premier temps de façon traditionnelle, comme un implant rigide. Cette phase de fonctionnement est par exemple celle de formation du cal dans le cas d'une greffe. Dans un deuxième temps, les moyens de blocage sont retirés et l'implant fonctionne selon l'invention, en exerçant entre les éléments auxquels il est relié une force proportionnelle à leur vitesse relative et donc fonction des contraintes exercées dans la greffe.

Egalement dans un mode de réalisation particulier, l'implant comprend des moyens de limitation de la course du dispositif amortisseur.

Ceci présente l'avantage de rendre l'implant rigide dans le cas où les contraintes atteignent une certaine limite. On est ainsi certain de ne pas se trouver dans la zone des contraintes de rupture.

Avantageusement, lesdits moyens de limitation de la course sont réglables.

La course du dispositif amortisseur peut ainsi être adaptée au patient et éventuellement augmentée progressivement tandis que la greffe se renforce.

Un implant selon l'invention peut être réalisé sous la forme d'un organe allongé tel qu'une vis, ou une broche telle qu'une broche de prothèse coxo-fémorale, ou encore le col, le corps ou la tête d'une prothèse fémorale, comprenant deux parties d'extrémités reliées par une partie médiane souple, la partie médiane comprenant deux chambres remplies de fluide hydraulique, situées de part et d'autre d'une fibre neutre et agencées l'une pour augmenter de volume, et l'autre pour diminuer de volume lorsque l'implant fléchit, lesdites chambres étant reliées par au moins un conduit calibré.

Ce mode de réalisation peut être utilisé pour relier ensemble les deux parties d'un os fracturé, par exemple le fémur au niveau du col ou de la diaphyse. La vis est mise en place de sorte que sa partie médiane se trouve à l'emplacement de la fracture, avec sa fibre neutre disposée autant que possible dans le plan des flexions maximales sous effort. Ainsi, après formation du cal, l'implant continuera à l'assister lors d'efforts soudains, le fluide hydraulique étant chassé de la chambre dont le volume diminue vers celle dont le volume augmente, à travers l'orifice calibré. En revanche, le cal reprendra seul les efforts statiques.

Plus particulièrement, lesdites parties d'extrémité peuvent être reliées par une paroi élastique délimitant lesdites chambres avec un soufflet périphérique.

On verra ci-après qu'il est souvent avantageux d'ajouter à la composante visqueuse du comportement de l'implant une composante élastique.

Ledit conduit calibré peut être réalisé sous la forme de perçages dans au moins une des parties d'extrémité.

On obtient ainsi une réalisation très compacte, bien adaptée à la réalisation de l'implant sous la forme d'une vis.

Avantageusement, on prévoit une vanne sur ledit conduit calibré.

Cette vanne peut tout d'abord être fermée pendant la durée de formation du cal. Ainsi l'implant est parfaitement rigide et se comporte donc comme une vis classique. La vanne est ensuite ouverte pour que l'implant fonctionne selon l'invention, avec sa fonction d'amortisseur. Une vanne à ouverture progressive permet en outre d'assurer la fonction de réglage du coefficient d'amortissement.

La commande de la vanne est de préférence logée à l'extrémité de l'implant la plus proche de la peau.

Ainsi, une intervention tout à fait bénigne permet de procéder à l'ouverture de la vanne et, éventuellement à son réglage.

Dans un mode de réalisation particulier, adapté notamment à la réalisation d'une greffe osseuse visant au remplacement d'une vertèbre lésée, le dispositif amortisseur comprend au moins une chambre formée de deux demi-chambres réunies par un soufflet, chacune des demi-chambres étant reliée à une des parties de l'implant, ladite chambre étant remplie d'un fluide hydraulique, et au moins un orifice calibré étant prévu dans une paroi de ladite chambre pour faire communiquer ladite chambre avec une autre chambre.

Cette disposition présente l'avantage d'être très compacte et d'éviter des coulissement relatifs de pièces mécaniques, avec les risques de fuite de fluide hydraulique qui en résultent. Toutefois, on pourra dans certains cas préférer un amortisseur traditionnel à cylindre et piston.

Plus particulièrement, lesdites demi-chambres peuvent être en forme de coupelles dont les ouvertures se font face.

Les moyens de blocage précités peuvent ici comprendre un organe de blocage allongé, amovible, engagé dans un pli dudit soufflet pour empêcher son écrasement.

L'organe de blocage allongé peut être réalisé de toute manière convenable, par exemple sous la forme d'une chaînette de billes métalliques.

Les moyens précités de limitation de la course peuvent comprendre une couronne de butée vissée sur une desdites demi-chambres et agencée pour coopérer avec un épaulement de l'autre demi-chambre pour s'opposer au rapprochement des deux demi-chambres au delà d'une certaine limite.

Ladite autre chambre peut être logée dans une jupe d'appui montée sur une desdites demi-chambre autour de l'orifice calibré, à l'opposé de l'autre demi-chambre.

Cette autre chambre peut en outre être également formée de deux demi-chambres réunies par un soufflet.

Dans un mode de réalisation préféré, l'implant selon l'invention comprend en outre des moyens élastiques entre lesdites deux parties.

Il est en effet souvent utile que la résistance au déplacement relatif des deux parties de l'implant soit non seulement proportionnelle à la vitesse de ce déplacement, mais également à la valeur de ce déplacement lui-même par rapport à une position nominale. On obtient ainsi une assistance de l'implant aux structures osseuses environnantes d'autant plus efficace qu'elles sont plus écartées de leur configuration normale.

Avantageusement, le coefficient d'élasticité est réglable.

On peut ainsi ajuster l'élasticité de l'implant et le rendre par exemple de plus en plus souple au fur et à mesure du rétablissement des structures environnantes.

Une telle élasticité peut être obtenue dans un implant comprenant deux chambres remplies de fluide hydraulique et réunies par un orifice calibré, l'une au moins des chambres contenant une ampoule de compression à pression ambiante à parois élastiques.

Lorsqu'un mouvement s'amorce entre les deux parties de l'implant, il se produit une variation de pression dans les chambres, et donc une réaction élastique de l'ampoule de compression.

La paroi de ladite ampoule peut posséder une élasticité progressive, l'une desdites chambres étant susceptible d'être reliée à une source de fluide sous pression.

On peut donc dans ce cas régler l'élasticité de l'implant en ajustant la pression dans les chambres.

Des moyens peuvent être prévus pour régler la section dudit orifice calibré.

En particulier, lesdits moyens de réglage de la section de l'orifice calibré peuvent comprendre une valve à pointeau à commande hydraulique.

La présente invention a également pour objet un implant du type rappelé ci-dessus, dans lequel le dispositif amortisseur comprend au moins deux chambres, notamment deux chambres basse pression dont les parois sont réalisées en matériau élastique, lesdites chambres étant reliées par au moins un conduit calibré et remplies de fluide hydraulique, et agencées pour subir une variation de pression différentielle lors d'un mouvement relatif desdits éléments.

Un tel agencement permet d'obtenir simplement des implants qui possèdent comme ci-dessus non seulement un comportement de résistance visqueuse, fonction de la vitesse, mais également de résistance élastique, fonction du déplacement.

Dans ce cas, le conduit peut avantageusement avoir sa section déterminée par la pression régnant dans une chambre haute pression susceptible de comprimer ce conduit.

Grâce à cet agencement, le coefficient d'amortissement de l'implant peut être très facilement réglé en ajustant la pression dans la chambre haute pression.

En particulier, une des chambres basse pression peut posséder une paroi de rigidité relativement faible par rapport à la rigidité des parois de l'autre chambre.

Cet agencement permet à l'implant de fonctionner même lorsque les deux chambres ne sont pas sollicitées de façon différentielle. Lors d'un mouvement créant une surpression, la pression augmente plus dans la chambre à paroi rigide, d'où résulte un écoulement du fluide hydraulique de cette chambre vers la chambre à paroi moins rigide, et donc un effet d'amortissement.

Dans un mode de réalisation particulier, une chambre haute pression et une chambre basse pression peuvent être reliées, dans le sens de la basse à la haute pression, par un clapet anti-retour.

On verra que cette disposition permet de faire augmenter la différence de pression entre les chambres haute et basse pression. Elle permet également de maintenir cette différence en dépit d'éventuelles fuites de la haute pression vers la basse pression.

Ledit clapet anti-retour peut comprendre un tuyau souple relié à la chambre basse pression, dont une extrémité libre est engagée dans une extrémité libre d'un tuyau relié à la chambre haute pression.

Un tel clapet est très peu encombrant et présente en outre l'avantage d'être d'autant mieux fermé que la différence de pression est plus élevée.

De préférence, on prévoit en outre que lesdites chambres sont reliées par une valve de régulation de pression en parallèle sur le clapet anti-retour.

On verra que de préférence ledit clapet anti-retour est disposé entre la chambre basse pression à rigidité élevée et la chambre haute pression.

Dans un mode de réalisation particulier, l'implant selon l'invention comprend une première chambre basse pression annulaire, et une deuxième chambre de révolution au centre de la première chambre, lesdits conduits calibrés étant formés radialement dans la paroi séparant les deux chambres.

Avantageusement, la paroi extérieure de la première chambre basse pression est relativement mince, et la paroi séparant les deux chambres basse pression est relativement épaisse.

Cet implant peut comprendre au moins une chambre haute pression annulaire formée dans l'épaisseur de la paroi séparant les deux chambres basse pression, et agencée pour comprimer lesdits conduits'calibrés.

Dans un autre mode de réalisation, ledit conduit est au moins partiellement réalisé sous la forme d'un tube en matériau élastique entouré par un tube sensiblement plus rigide, lesdits tubes étant reliés annulairement à leurs extrémités, le volume compris entre les deux tubes formant une chambre haute pression.

Dans un autre mode de réalisation particulier, l'implant est sensiblement en forme de disque, comportant une pluralité de chambres basse pression en forme de secteurs, reliées par lesdits conduits calibrés, eux-mêmes alternés dans l'épaisseur du disque avec lesdites chambres haute pression.

Avantageusement, l'implant selon l'invention comprend des moyens de réglage de la distance à laquelle il relie lesdits éléments.

Un tel agencement permet en particulier de soulager d'éventuelles douleurs postopératoire en ajustant cette distance convenablement. Il est également particulièrement intéressant dans le cas de prothèses destinées à des enfants en cours de croissance.

Lesdits moyens de réglage peuvent comprendre un soufflet agencé pour recevoir un fluide hydraulique, et des moyens de connexion dudit soufflet à une source de fluide sous pression.

L'invention à également pour objet une paire d'implants tels que décrits ci-dessus, la chambre basse pression de chacun des implants étant reliée par ledit clapet anti-retour à la chambre haute pression de l'autre implant.

Une telle paire d'implants peut notamment être prévue en cas de greffe de la colonne vertébrale, pour assister la greffe en cas de flexion latérale.

Plus généralement, une paire d'implants selon l'invention peut comporter des moyens d'auto-adaptation aux mouvements du porteur des implants.

On a jusqu'à présent décrit des implants de consolidation osseuse réalisés selon l'invention. On va voir maintenant que l'invention est également bien adaptée à la réalisation d'implants articulaires.

Dans ce cas, un implant tel que décrit ci-dessus a chacune de ses parties articulée sur l'autre.

Plus particulièrement, lesdites parties peuvent posséder des surfaces complémentaires en appui l'une sur l'autre, formant une articulation à rotule.

Un tel l'implant articulaire peut en particulier comprendre un pivot solidaire d'un desdites parties et logé dans un espace formé entre une pluralité de chambres basse pression en forme de secteurs, solidaires de l'autre partie de l'implant et reliées par lesdits conduits calibrés, eux-mêmes alternés avec des chambres haute pression.

Ces modes de réalisation sont bien adaptés comme disques intervertébraux.

Dans une application particulière à une articulation coxo-fémorale, l'implant selon l'invention comprend une sphère creuse d'articulation, dont la paroi est ouverte pour permettre le passage de l'extrémité d'une broche de fixation, ledit dispositif amortisseur étant disposés dans ladite sphère entre la paroi de cette dernière et l'extrémité de la broche de fixation.

Plus particulièrement, ledit dispositif amortisseur peut comprendre un organe d'extrémité de la broche de fixation agencé pour coulisser dans une découpe d'une cloison interne à la sphère, ladite cloison délimitant deux chambres dans la sphère, et au moins un orifice calibré étant formé dans ledit organe d'extrémité entre les deux dites chambres.

Dans un autre mode de réalisation, ledit dispositif amortisseur peut comprendre un organe d'extrémité de la broche de fixation disposé entre deux organes d'amortissement comprenant chacun au moins deux chambres basse pression dont les parois sont réalisées en matériau élastique, lesdites chambres étant reliées par au moins un conduit calibré et remplies de fluide hydraulique, et agencées pour subir une variation de pression différentielle lors d'un mouvement relatif de la sphère et de la broche de fixation.

L'invention a également pour objet une paire d'implants tels que décrits ci-dessus, utilisée notamment dans le cadre d'une arthrodèse de la colonne vertébrale, chacun des implants étant associé mécaniquement en série à une broche de liaison de type connu.

Plus particulièrement, chacun des implants peut comprendre des moyens de réglage de la distance à laquelle il relie lesdits éléments.

Il est ainsi possible, à l'aide d'une telle paire d'implants, non seulement de réaliser l'arthrodèse, mais encore de régler l'angle et la distance entre les deux parties de la colonne vertébrale reliées par la prothèse.

Bien entendu, de tels moyens de réglage pourraient être utilisés indépendamment des moyens relatifs à la viscosité et à son réglage.

Dans un mode de réalisation particulier, lesdits moyens de réglage comprennent, pour chaque implant, un organe gonflable tel qu'un soufflet, agencé pour recevoir un fluide hydraulique, et des moyens de connexion dudit soufflet à une source de fluide sous pression.

Ladite source de fluide sous pression peut comprendre un réservoir de fluide haute pression.

Avantageusement, ledit réservoir haute pression est commun aux deux implants, chaque organe gonflable est également connecté à un réservoir basse pression, et une cellule de recharge gonflable est associée mécaniquement en série à chaque broche, chaque cellule de recharge étant connectée aux réservoirs haute et basse pression par deux clapets anti-retour, l'un permettant un écoulement de fluide du réservoir basse pression à la cellule de charge, et l'autre permettant un écoulement de fluide de la cellule de charge au réservoir haute pression.

On verra qu'un tel agencement permet de réaliser une pompe actionnée par les mouvements propres du porteur des implants.

Egalement dans un mode de réalisation particulier, la paire d'implants précitée est formée d'implants dans lesquels le dispositif amortisseur comprend au moins deux chambres, lesdites chambres étant reliées par au moins un conduit calibré et remplies de fluide hydraulique, et agencées pour subir une variation de pression différentielle lors d'un mouvement relatif desdits éléments, ledit conduit calibré ayant sa section déterminée par la pression régnant dans une chambre haute pression, et les chambres haute pression des implants sont connectées au réservoir haute pression par une vanne commandable

L'invention a également pour objet un implant squelettique tel que décrit ci-dessus, notamment appartenant à une paire d'implants, comportant des capteurs de grandeurs physiques, notamment de pression, alimentés électriquement et commandés extracorporellement de manière non invasive, et agencés pour transmettre leurs informations à des moyens d'affichage.

Plus particulièrement, cet implant peut aussi comporter des actionneurs de réglage également alimentés électriquement et commandés extracorporellement de manière non invasive.

L'alimentation et la télécommande peuvent se faire par radiofréquences ou par ultrasons. Il est ainsi possible d'effectuer toutes les opérations de réglage et d'adaptation en postopératoire, par des moyens totalement non invasifs.

On décrira maintenant, à titre d'exemple non limitatif, des modes de réalisation particulier de l'invention, en référence aux dessins schématiques annexés dans lesquels:
- les figures 1a et 1b illustrent schématiquement le principe de l'invention appliqué à la formation d'un cal après la fracture d'un os long;
- la figure 2 illustre schématiquement un procédé de reconstruction fémorale après fracture du col;
- la figure 3 montre une vis selon l'invention susceptible d'être utilisée pour la mise en oeuvre du procédé de la figure 2;
- la figure 4 est une vue à plus grande échelle du détail IV de la figure 3;
- la figure 5 est une vue en coupe axiale à encore plus grande échelle du détail IV;
- la figure 6 est une vue en coupe axiale schématique illustrant le fonctionnement de cette vis;
- la figure 7 est une vue en coupe axiale schématique d'un dispositif amortisseur susceptible d'être utilisé dans un implant selon l'invention;
- la figure 8 est une vue partielle du dispositif de la figure 7 représentant un certain nombre de perfectionnements;
- la figure 9 est une vue partielle en coupe selon la ligne IX-IX de la figure 8;
- la figure 10 est une vue en perspective d'organes des figures 8 et 9;
- les figures 11a et 11b sont également des vues partielles du dispositif de la figure 7 représentant d'autres perfectionnements, dans deux phases successives d'utilisation du dispositif;
- la figure 12 est une vue en élévation, partiellement en coupe, d'un implant rachidien réalisé selon l'invention;
- la figure 13 représente l'utilisation de deux implants selon l'invention dans le cadre d'une arthrodèse rachidienne;
- la figure 14 montre en détail, en coupe axiale, les implants de la figure 13, en coupe selon la ligne XIV-XIV de la figure 15, et leurs interconnexions;
- la figure 15 est une vue en coupe transversale selon la ligne XV-XV de la figure 14;
- la figure 16 est une modélisation du dispositif des figures 13 à 15;
- les figures 17a à 17d illustrent le fonctionnement des dispositifs des figures 13 à 16;
- la figure 18 représente en vue de dessus, partiellement en coupe transversale, un autre mode de réalisation d'une prothèse rachidienne selon l'invention;
- la figure 19 est une vue en coupe selon la ligne XIX-XIX de la figure 18;
- la figure 20 est une vue en coupe selon la ligne XX-XX de la figure 18;
- la figure 21 est une vue en coupe selon la ligne XXI-XXI de la figure 18;
- la figure 22 illustre l'interconnexion des chambres de la prothèse de la figure 18;
- la figure 23 montre un autre montage possible de deux implants tels que ceux de la figure 14;
- la figure 24 illustre une variante de la figure 23;
- la figure 25 est une vue schématique en coupe axiale d'une application de l'invention à une prothèse intervertébrale;
- la figure 26 montre l'implantation de la prothèse de la figure 25;
- la figure 27 est une vue en coupe axiale d'un "ligament" de la figure 26;
- la figure 28 représente en vue schématique de dessus, un mode de réalisation d'une prothèse intervertébrale selon l'invention;
- la figure 29 est une vue en coupe selon la ligne XXIX-XXIX de la figure 28;
- la figure 30 est une vue en coupe selon la ligne XXX-XXX de la figure 28;
- la figure 31 est une vue en coupe selon la ligne XXXI-XXXI de la figure 28;
- la figure 32 est une vue en perspective montrant plus en détail l'implantation de la prothèse intervertébrale des figures 28 à 31;
- la figure 33 est une vue en perspective d'un clapet anti-retour susceptible d'être utilisé dans un implant selon l'invention;
- la figure 34 en est une vue en coupe axiale;
- les figures 35 et 36 sont des vues en perspective d'une prothèse coxo-fémorale réalisée selon l'invention;
- la figure 37 en est une vue en coupe transversale schématique;
- la figure 38 est une vue en coupe de la tête de la prothèse des figures 35 à 37;
- la figure 39 est une vue partielle éclatée de la prothèse des figures 35 à 38;
- la figure 40 montre un autre implant susceptible d'être utilisé à la place de ceux de la figure 13;
- la figure 41 est une vue en perspective d'un organe de l'implant de la figure 40;
- la figure 42 en est une vue en coupe axiale;
- la figure 43 est une vue similaire à la figure 41 d'un autre mode de réalisation;
- la figure 44 est une vue en coupe axiale de ce mode de réalisation;
- la figure 45 illustre le fonctionnement des organes des figures 41 à 44;
- la figure 46 en illustre un autre mode de fonctionnement;
- la figure 47 est une vue schématique de l'ensemble du circuit hydraulique d'une arthrodèse de la colonne vertébrale utilisant des implants du type de celui de la figure 40;
- la figure 48 représente le montage des éléments de cette arthrodèse; et
- la figure 49 est un schéma électrique de la commande d'un implant selon l'invention.

Les figures la et 1b représentent un os long 1 dans la partie médiane 2 duquel a été formée une greffe 3, entre deux éléments d'extrémités 4 et 5.

Un implant désigné d'une manière générale par la référence 6 est prévu pour consolider la greffe 3. A cet effet, l'implant 6 comporte deux parties 7 et 8 vissées chacune dans un des éléments osseux 4 et 5 respectivement. Selon l'invention, les extrémités en vis-à-vis des parties 7 et 8 sont reliées par un dispositif amortisseur 9.

L'amortisseur 9 est composé de façon connue d'un cylindre 10 formant deux chambres fermées 11 et 12 respectivement, séparées par une tête 13 de piston, montée coulissante dans le cylindre. A cet effet, le cylindre 10 est rempli d'un fluide hydraulique, susceptible d'être laminé au niveau d'un orifice calibré (non représenté) formé dans la tête 13 de piston.

Des moyens de tout type connu, également non représentés, sont prévus pour régler le coefficient d'amortissement, par exemple des moyens de réglage de la section de l'orifice calibré. De tels moyens de réglage sont présents dans tous les modes de réalisation décrits ci-dessous, même dans les cas où ils ne seront pas mentionnés.

L'extrémité de la partie 7 de l'implant est reliée à une tige 14 de piston, fixée à la tête 13 et traversant un des flasques d'extrémité du cylindre. L'extrémité de la partie 8 de l'implant est reliée au cylindre 10.

En outre, une liaison rigide amovible 15 relie à l'origine les extrémités des parties 7 et 8 de l'implant, mettant ainsi l'amortisseur 9 hors service.

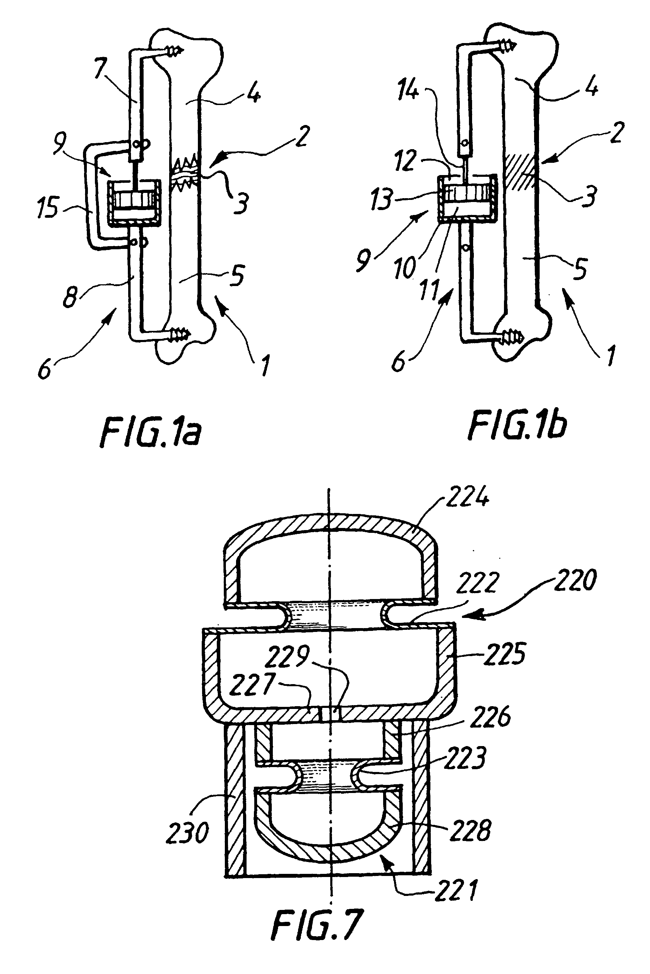

L'implant 6 est mis en place avec sa liaison 15 (figure 1a), qui est maintenue tant qu'un cal ne s'est pas formé au niveau de la greffe. Du fait de cette liaison, l'implant se comporte comme un implant classique, reprenant les efforts tant statiques que dynamiques. Lorsque le cal est formé (figure 1b), la liaison 15 est retirée ou inhibée.

L'amortisseur exerçant entre les parties 7 et 8 de l'implant 9 une force proportionnelle à la vitesse relative de ces deux parties, l'os partiellement reconstitué reprendra intégralement les efforts statiques. En revanche, il sera d'autant plus assisté par l'amortisseur à la reprise des efforts dynamiques que ceux-ci seront importants et provoqueront donc des vitesses de déformation importantes, ici en traction et en compression.

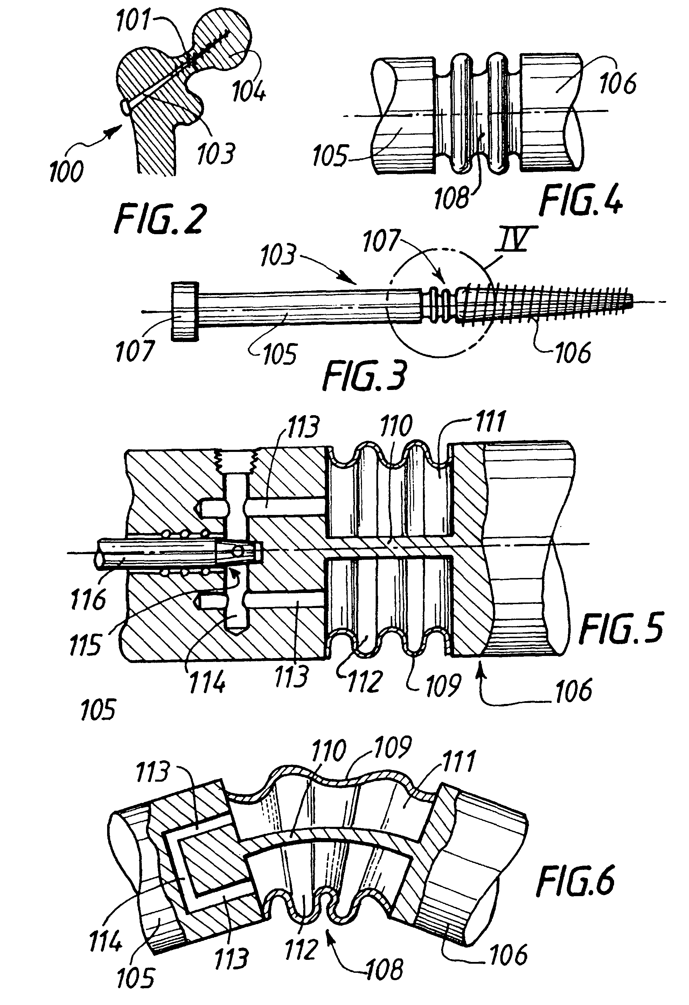

Les figures 2 à 6 montrent une application du principe exposé en référence aux figures 1.

On y voit la partie haute d'un fémur 100 ayant subi une fracture au niveau du col 101. De façon connue, une vis 103 est introduite dans le corps du fémur et dans sa tête 104, pour maintenir cette dernière jusqu'à formation d'un cal et soudure des deux parties séparées par la fracture.

La vis 103 est réalisée en deux parties rigides sensiblement cylindriques, à savoir respectivement un corps 105, partie de la vis la plus proche de la peau, et une pointe filetée 106, partie la plus éloignée. La partie la plus proche de la peau comporte à son extrémité extérieure une tête 107 utilisée pour le vissage de la vis 103. Les deux parties d'extrémité sont reliées par une partie médiane souple 108.

La partie médiane 108 est formée d'un soufflet annulaire 109 reliant les périphéries des extrémités internes du corps 105 et de la pointe 106. Une lame élastique plane 110 relie également lesdites extrémités, cette lame contenant leur axe et, ainsi, permettant un mouvement de pivotement relatif entre les parties 105 et 106 dans un plan perpendiculaire à la lame, tout en interdisant un tel mouvement dans le plan de la lame. La lame 110 forme ainsi une fibre neutre dans un mouvement de rotation entre les parties 105 et 106 autour d'un axe perpendiculaire à l'axe de ces parties et contenu dans le plan de la lame 110.

Ainsi, le soufflet 109 et la lame 110 délimitent deux chambres 111 et 112 situées de part et d'autre de la fibre neutre. Lors d'une flexion de la vis, entraînant une rotation relative de ses parties 105 et 106 autour de l'axe précité, l'une des chambres (111 sur la figure 6) voit son volume augmenter, tandis que l'autre (112) voit son volume diminuer.

Les chambres 111 et 112, ainsi que leurs conduits de communication décrits ci-après, sont remplies d'un fluide hydraulique.

Des perçages 113 parallèles à l'axe de la vis communiquent chacun avec une des chambres 111 et 112 et communiquent entre eux par un perçage transversal 114. Une vanne 115 (non représentée à la figure 6) montée sur le perçage 114 permet d'ouvrir ou de fermer la communication entre les chambres 111 et 112, ainsi que de régler la section du passage entre ces chambres.

La tige de commande 116 de la vanne 115 est coaxiale au corps 105 de la vis, et sa tête de commande, par exemple elle-même une vis, est incluse dans la tête 107 de la vis. Elle se trouve donc vers l'extérieur du corps du patient où la vis est implantée.

La vis 103 est mise en place de façon connue, mais de manière que sa partie médiane 108 soit située au niveau de la fracture. En outre, la vis est immobilisée en rotation axiale de manière que le plan de la lame 110 se trouve perpendiculaire au plan de la figure 2, de manière que puisse se produire une rotation relative des parties 105 et 106 de la vis autour d'un axe perpendiculaire à ce plan et passant au niveau de la partie 108.

Lors de l'implantation, la vanne 115 est fermée. Aucune communication n'est donc permise entre les chambres 111 et 112, de sorte que la vis est parfaitement rigide tout le temps que dure la formation du cal. Une fois le cal formé, une intervention mineure permet d'accéder à la tête de commande de la vanne 115, et donc d'ouvrir cette vanne et de régler son ouverture, et, par conséquent, le coefficient d'amortissement de l'implant.

Les figures 1 illustrent l'utilisation d'un amortisseur classique. On sera toutefois généralement amené à utiliser des dispositifs amortisseurs mieux adaptés à l'usage particulier que l'on veut en faire.

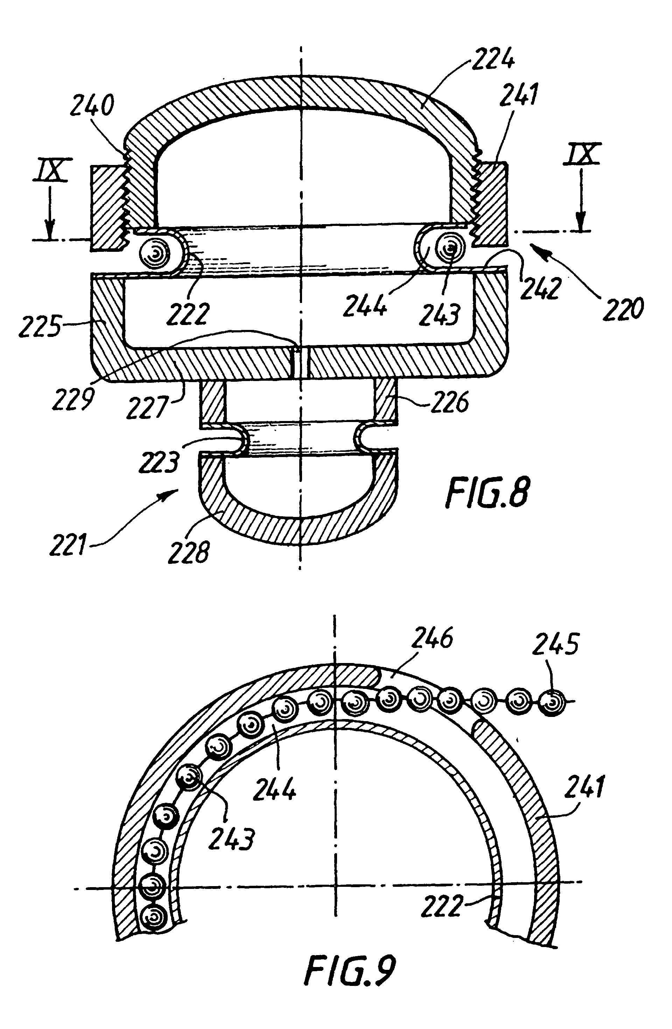

Ainsi, la figure 7 montre un dispositif amortisseur dans lequel chacune des deux chambres 220 et 221 ont un volume variable dû à la présence d'un soufflet 222 et 223 respectivement.

La chambre 220 est formée de deux demi-chambres en forme de coupelles 224 et 225 dont les ouvertures se font face. Ces coupelles sont reliées par le soufflet 222.

De même, la chambre 221 est formée de deux demi-chambres dont l'une est constituée d'un cylindre 226 soudé à l'extérieur du fond 227 de la coupelle 225 et l'autre est une coupelle 228 dont l'ouverture fait face au cylindre 226. Le cylindre 226 et la coupelle 228 sont reliés par le soufflet 223.

Chacun des soufflets est ici réalisé sous la forme d'un secteur de tore en tôle métallique soudé le long de chacun de ses bords, le long des bords des coupelles 224 et 225 en ce qui concerne le soufflet 222, et le long du bord libre du cylindre 226 et du bord de la coupelle 228 en ce qui concerne le soufflet 223.

Les chambres 220 et 221 sont remplies d'un fluide hydraulique et communiquent par un orifice calibré 229 percé dans le fond 227 de la coupelle 225 et débouchant dans le cylindre 226. Ainsi, lorsqu'un effort de compression est exercé sur les coupelles 224 et 225 de la chambre 220, tendant à les rapprocher, cette chambre voit son volume diminuer tandis que le fluide traverse l'orifice 229 et est refoulé dans la chambre 221 dont le volume augmente. L'effort s'opposant au rapprochement des coupelles 224 et 225 est proportionnel à la vitesse de rapprochement, en supposant que les soufflets n'exercent aucun effort, notamment de nature élastique.

Lorsque l'effort de compression cesse, les soufflets ramènent les chambres 220 et 221 à leur configuration d'origine.

Dans le mode de réalisation représenté, la chambre 221 est logée dans une jupe d'appui cylindrique 230 de transmission d'effort, soudée sur le fond 227 de la coupelle 225 autour du cylindre 226. Les efforts sont dans ce cas transmis au dispositif amortisseur par l'intermédiaire de la coupelle 224 et de la jupe 230, ces deux organes reliant les deux parties de l'implant.

Dans les figures 8 à 10, la coupelle 224 comporte, dans une zone cylindrique, un filetage extérieur 240 sur lequel est vissée une couronne de butée 241 munie d'un filetage intérieur coopérant. Une extrémité de la couronne 241 fait face à un épaulement 242 de la coupelle 225, ici le bord de cette dernière coupelle sur lequel est soudé un bord du soufflet 222.

En outre, une chaînette de billes 243 est engagée dans l'espace annulaire 244 délimité par le pli du soufflet 222, y compris le bord de la coupelle 224, et le bas de la couronne 241. Les billes de la chaînette ont ici un diamètre sensiblement égal à la distance à l'équilibre entre les bords en vis-à-vis des coupelles 224 et 225. Une extrémité 245 de cette chaînette sort de l'espace annulaire 244 par une encoche 246 formée dans la paroi latérale de la butée 241, en vis-à-vis de l'épaulement 242.

Lors de la mise en place de l'implant, puis dans la phase ultérieure de consolidation, la chaînette 243 empêche l'écrasement du soufflet 222 de sorte que, en compression, le dispositif se comporte sensiblement comme un implant rigide traditionnel. Si la chaînette possède un diamètre inférieur à l'épaisseur de l'espace 244, le dispositif possède partiellement, dès cette phase, une fonction d'amortisseur.

Après consolidation, la chaînette 243 est retirée par traction sur son extrémité 245, le dispositif fonctionnant alors totalement selon le principe d'amortisseur de l'invention. Toutefois, une limite d'écrasement est obtenue par la venue en butée du bord de la couronne 241 avec l'épaulement 242 de la coupelle 225. Le dispositif fonctionne alors de nouveau, en compression, comme un implant rigide.

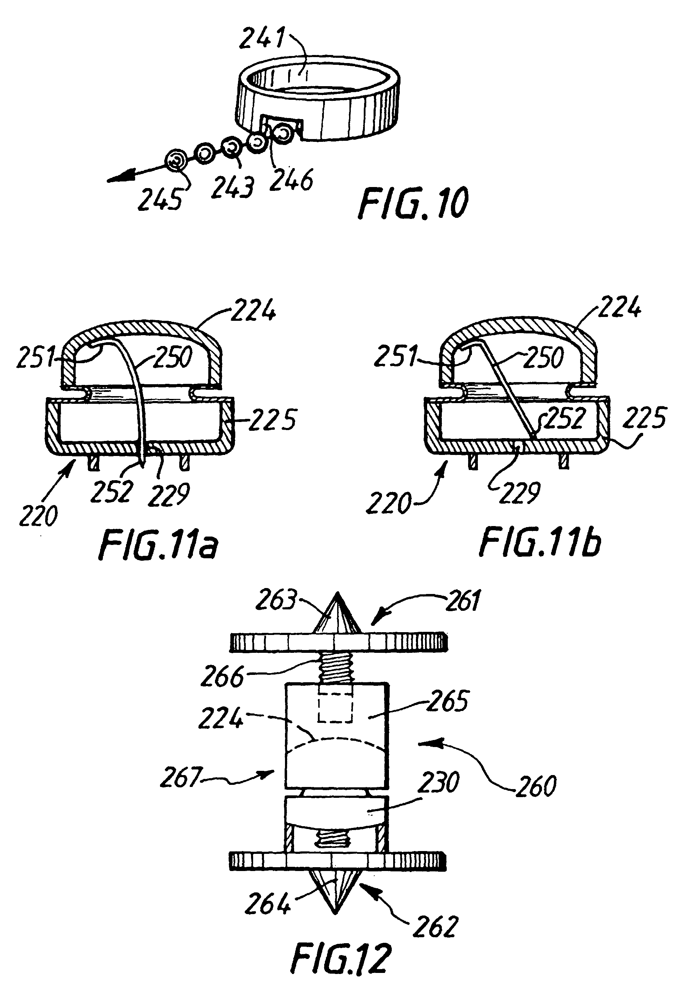

Les figures 11a et 11b illustrent un mode de réalisation alternatif au système de blocage de la chaînette 243 des figures 8 à 10. Une aiguille élastique 250 a ici une de ses extrémités 251 soudée à l'intérieur de la coupelle 224 de la chambre 220. A l'origine, l'autre extrémité 252 de l'aiguille 250 est engagée dans l'orifice calibré 229 qu'elle obstrue. Du fait de l'incompressibilité du fluide hydraulique que contiennent les chambres du dispositif, celui-ci est parfaitement rigide.

Lorsque l'on souhaite faire fonctionner le dispositif conformément à l'invention, il suffit d'écarter convenablement les coupelles 224 et 225 l'une de l'autre de manière à dégager l'extrémité 252 de l'aiguille 250 de l'orifice 229. Cet orifice est alors libre de donner passage au fluide hydraulique et ne peut en aucun cas être rebouché par l'aiguille 250.

L'implant rachidien 260 de la figure 12 comporte deux parties d'extrémité 261 et 262 munies de cônes d'accrochage 263 et 264 respectivement. La partie 261 comprend en outre une douille 265 reliée au cône d'accrochage par une vis de réglage de longueur 266.

Un dispositif amortisseur 267, ici du type de ceux décrits en référence aux figures 7 à 11, a sa coupelle 224 solidaire de la douille 265 et sa jupe 230 solidaire de la partie d'extrémité 262.

Un tel implant peut être utilisé lors d'opérations de reconstitution du fonctionnement ou de l'intégrité de la colonne vertébrale. Les vertèbres conservent au moins en partie leurs fonctions structurelles en cas de sollicitations statiques. En revanche, l'implant est d'autant plus actif que les sollicitations dynamiques sont importantes.

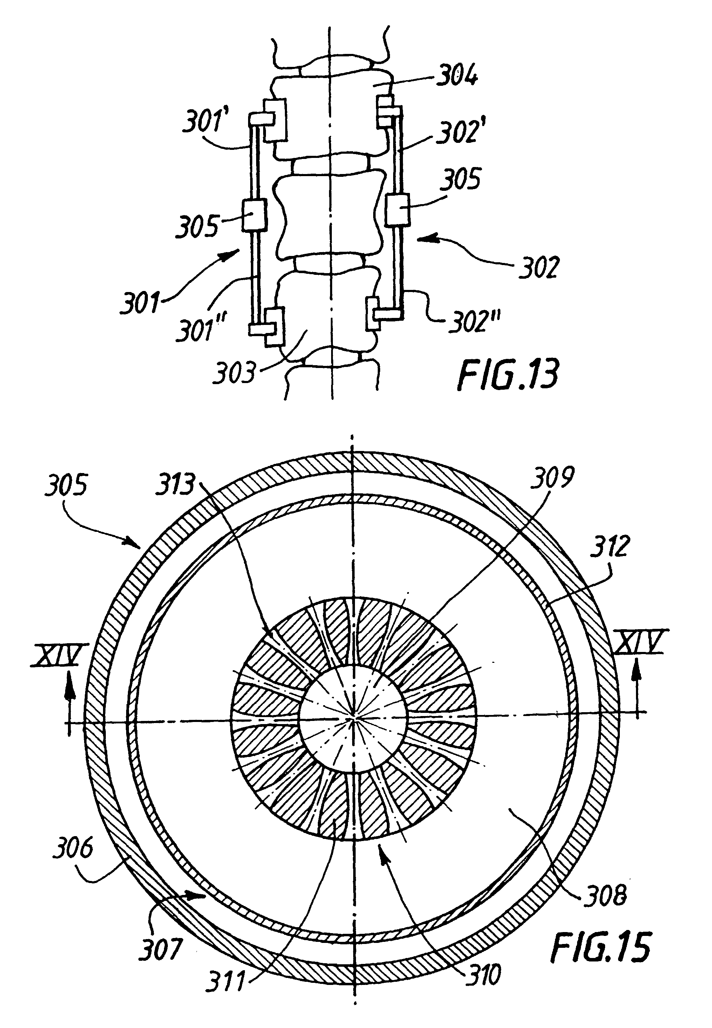

On voit maintenant sur la figure 13 une arthrodèse de la colonne vertébrale dans laquelle deux broches 301 et 302 sont ancrées dans deux vertèbres 303 et 304, de manière à, de façon connue, assurer une liaison entre ces deux vertèbres. Les broches 301 et 302 sont ici réalisées chacune en deux parties, 301', 301", et 302', 302" respectivement, chacune de ces parties étant reliée à une de ses extrémités à une des vertèbres 303 et 304, et à son autre extrémité à l'autre partie, par l'intermédiaire d'un dispositif 305 selon l'invention.

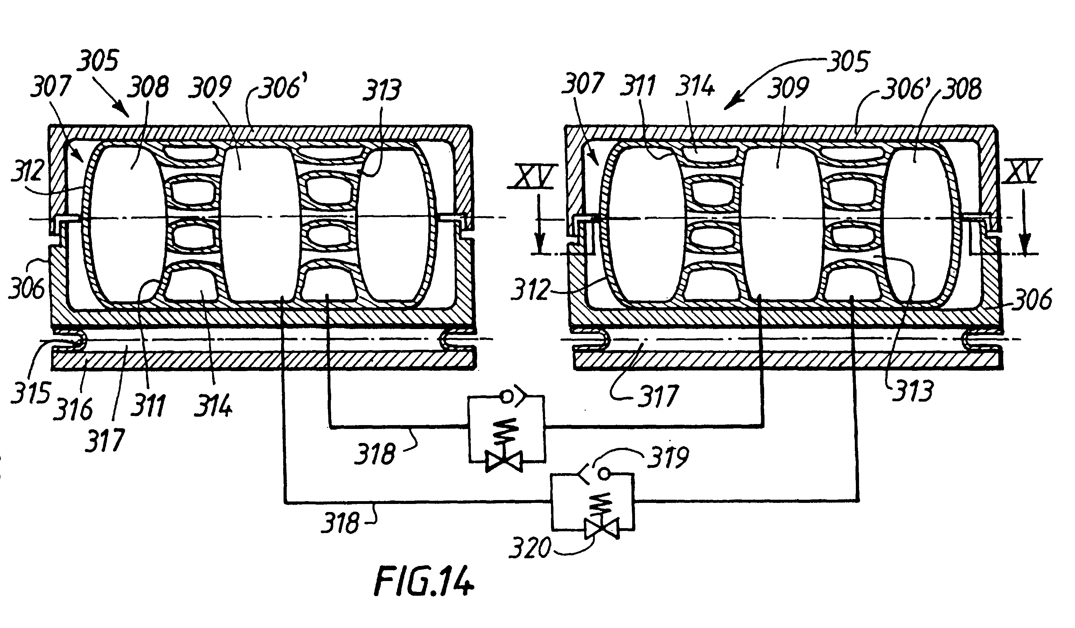

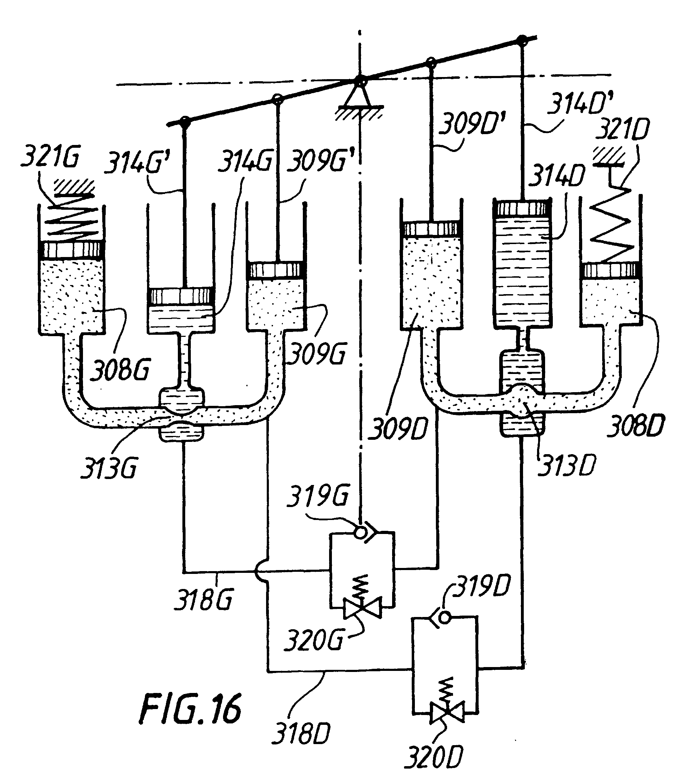

Un exemple d'un tel dispositif est décrit en référence aux figures 14 et 15, en coupe respectivement transversale et axiale.

L'implant 305 comprend un boîtier constitué de deux demi-boîtiers 306 et 306', respectivement inférieur et supérieur. A l'intérieur de ce boîtier est disposée une structure alvéolaire 307, notamment en silicone, assurant à la fois les fonctions de ressort et d'amortisseur entre les deux demi-boîtiers 306 et 306'.

La structure 307 comprend une chambre extérieure torique 308 et une chambre centrale 309 sensiblement cylindrique, ces deux chambres étant séparées par une cloison 310. La cloison 310 est constituée de la manière suivante.

Elle forme une paroi épaisse 311 de faible élasticité (de forte rigidité élastique) par rapport à la paroi extérieure 312 de la chambre extérieure 308. Dans cette paroi 311 sont ménagés des conduits radiaux 313 faisant communiquer les chambres 308 et 309. Entre les conduits 313 sont formées des chambres annulaires 314 communiquant librement entre elles, les conduits 313 et les chambres 314 étant répartis en nappes perpendiculaires à l'axe de la prothèse.

Le demi-boîtier inférieur 306 est ici relié par un soufflet 315 à une embase 316. Des moyens non représentés permettent d'injecter du fluide hydraulique sous pression dans la chambre 317 délimitée par le fond du demi-boîtier 306, le soufflet 315, et l'embase 316. Il est ainsi possible de régler l'épaisseur axiale de l'implant 305.

Bien évidemment, un réglage différentiel des deux soufflets 315 permet un redressement des vertèbres de la figure 13.

En prévoyant une gamme de réglage suffisante autorisant une vairation importante de la longueur du dispositif, il devient possible de réaliser un fixateur interne réglable, permettant la réparation de vertèbres sans obligation de fusion osseuse des articulations de ces vertèbres.

D'autres moyens non représentés, mais constitués pour l'essentiel de sites d'injection de fluide ou de boutons de réglage implantés sous la peau du patient, permettent d'ajuster la pression dans les chambres 308 et 309 d'une part, et 314 d'autre part, la pression étant en effet égale, à l'équilibre, dans les chambres basse pression 308 et 309, et inférieure à la pression dans la chambre haute pression 314.

La figure 14 montre, en outre, que la chambre basse pression centrale 309 de chaque prothèse 305 est reliée à la chambre haute pression de l'autre prothèse par des conduits 318 équipés de la manière que l'on va maintenant décrire.

Sur chaque conduit 318 est monté un clapet, anti-retour 319 susceptible de s'ouvrir d'une chambre basse pression 309 vers la chambre haute pression correspondante 314, lorsque la pression dans la chambre 309 devient supérieure à celle dans la chambre 314. En outre, une valve régulatrice de pression 320 est montée en parallèle du clapet 319, cette valve étant tarée de manière connue pour établir une pression différentielle prédéterminée entre les chambres basse pression 309 et haute pression 314.

On observera que les conduits entre les chambres basse pression pourraient en variante être disposées à l'extérieur du boîtier. Un mode de réalisation consiste à réaliser un tel conduit au moins partiellement sous- la forme d'un cathéter en matériau élastique entouré par un tube sensiblement plus rigide, le cathéter et le tube étant reliés annulairement à leurs extrémités, par exemple par collage ou par soudage. La chambre haute pression est alors constituée par le volume compris entre le cathéter et le tube.

On se référera maintenant à la figure 16, dans laquelle on a repris les même références que sur les figures 14 et 15, assorties des lettres D et G pour les implants droite et gauche respectivement (vue de l'arrière du patient porteur des prothèses).

Sur la figure 16, les différentes chambres sont assimilées à des vérins dont le corps constituerait la chambre proprement dite. On considère que ces corps de vérin sont fixes, c'est à dire que l'on suppose fixes les parties inférieures 301' et 302' des broches 301 et 302, les embases 316, et les demi-boîtiers inférieur 306.

Les pistons 309D' et 309G' et 314D' et 314G', illustrent les efforts exercés sur les chambres correspondantes par les demi-boîtiers supérieurs 306' lorsque le patient se penche latéralement, à gauche sur la figure 16 s'il est vu de dos. En ce qui concerne les vérins 308D et 308G, seule est modélisée l'élasticité des parois, par les ressorts 321 D et 321G, les variations de pression dans les chambres 308 ne résultant en pratique, du fait de cette élasticité, que des mouvements de fluide à travers les conduits 313.

Enfin, ces conduits 313 sont modélisés par des restrictions souples plus ou moins comprimées par le fluide contenu dans les chambres haute pression 314.

On va maintenant considérer le comportement de l'implant gauche, comprimé lors du mouvement. Du fait de l'épaisseur de la paroi 311 et donc de son peu d'élasticité, la chambre basse pression centrale 309 ne pourra que très légèrement augmenter en diamètre pour compenser sa diminution de hauteur. Le fluide qu'elle contient va donc être expulsé par les conduits 313 vers la chambre basse pression périphérique 308. On obtient donc la fonction d'amortisseur souhaitée.

Par ailleurs, l'élasticité de la paroi 312, modélisée par le ressort 321 de la figure 16, va simultanément avoir tendance à s'opposer au gonflement de la chambre 308, et donc à la pénétration du fluide dans cette chambre. On obtient ainsi la fonction de résistance élastique.

On observera que, lors du rapprochement des demi-boîtiers gauche 306 et 306', les chambres haute pression 314 gauche se rapprochent aussi, ce qui a pour effet, en réduisant les sections des conduits 313, d'augmenter le coefficient d'amortissement.

Du coté droit où les demi-boîtiers 306 et 306' ont au contraire tendance à s'écarter, les mouvements de fluide auront lieu en sens inverse. Du fait de la rigidité de ses parois, la section transversale de la chambre 309 va peu varier. Mais comme elle va voir sa hauteur, et donc son volume, augmenter, du fluide va y pénétrer en provenant de la chambre 308 à travers les conduits 313. Ceux-ci auront leurs sections augmentées, ce qui aura pour effet de diminuer, de ce coté, le coefficient d'amortissement et donc de compenser son augmentation de l'autre coté.

Le comportement élastique résultera pour l'essentiel de la traction exercée sur les parois latérale de la chambre 309.

On constate par conséquent que, dans le mouvement du patient, l'implant prend en charge essentiellement les déformations de forte amplitude ou à grande vitesse. Mais c'est la greffe osseuse qui reprendra les charges statiques modérées ou les charges résultant de mouvements relativement lents. Il en résultera une diminution des risques d'ostéoporose.

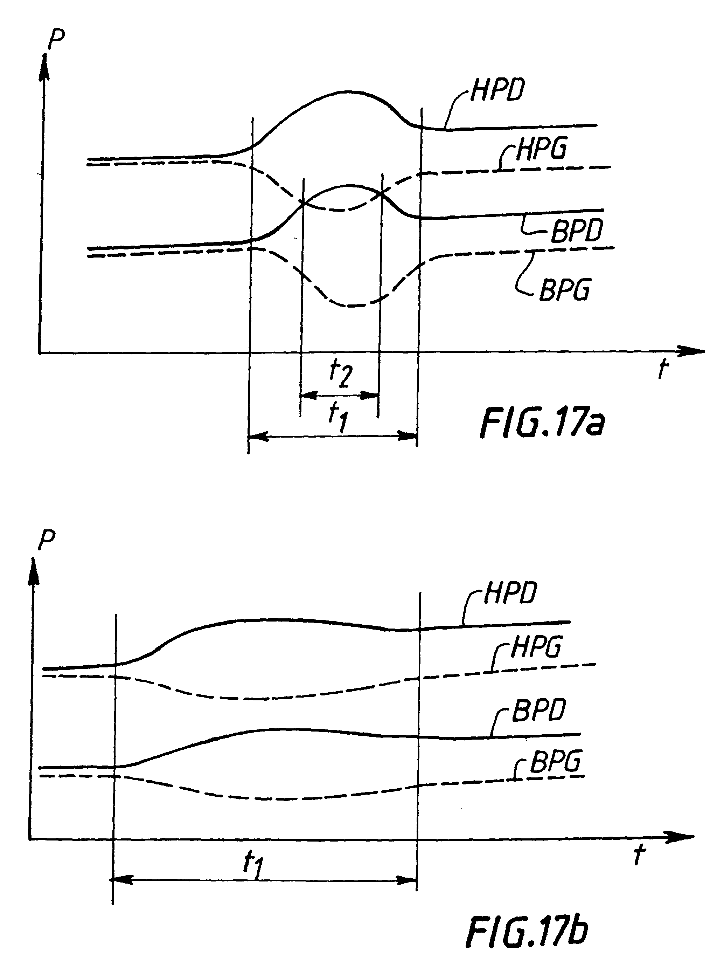

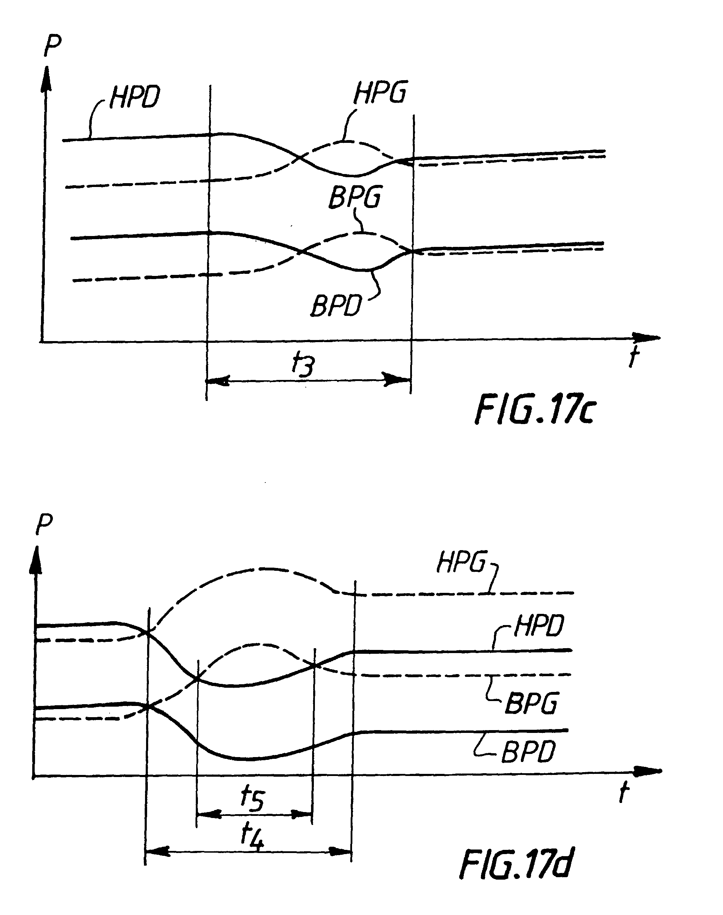

On va voir maintenant en référence aux figures 17a à 17d comment est régulée la pression différentielle entre les chambres haute et basse pression, essentielle au maintien des caractéristiques d'amortissement de l'implant. Ces figures illustrent les pressions dans les chambres basse pression centrale et haute pression en fonction du temps.

On supposera, en référence aux figures 17, que le patient successivement se penche rapidement vers sa droite (figure 17a), ou lentement (figure 17b), se redresse (figure 17c), et penche de nouveau, mais vers sa droite (figure 17d). Les lignes continues (HPD et BPD) concernent l'implant droit, et les lignes interrompues (HPG et BPG) l'implant gauche.

Lors d'un mouvement rapide, on observe un important pic de pression, positif du coté de l'inclinaison, négatif de l'autre, aussi bien dans les chambres haute pression que dans les chambres basse pression. On verra ci-après pourquoi en ce qui concerne les chambres haute pression. En ce qui concerne les chambres basse pression, ceci est dû à la rigidité des parois des chambres 309 et à l'effet d'amortissement des conduits 313, qui ralentissent l'écoulement de fluide des chambres 309 aux chambres 308.

On voit sur les figures 17a et 17b le temps t₁ que dure le mouvement du patient d'inclinaison vers la droite et pendant lequel les pressions varient, puis se stabilisent. Les pressions respectives étaient sensiblement égales avant le mouvement, par raison de symétrie, mais en fin de mouvement les pressions sont évidemment supérieures à droite qu'à gauche.

Lorsque le mouvement est suffisamment rapide, il existe une période t₂ incluse dans t₁ pendant laquelle la pression dans la chambre basse pression droite 309 devient supérieure à la pression dans la chambre haute pression gauche 314. Le clapet anti-retour 319G s'ouvre donc et permet le passage de fluide des chambres basse pression droite à la chambre haute pression gauche. Simultanément, la valve 320G de régulation de pression permet un écoulement en sens inverse de manière à éviter que la pression différentielle entre la haute et la basse pression dépasse la valeur de consigne.

On rétablit donc ainsi, si besoin était pour quelle que raison que ce soit, la pression différentielle prédéterminée par la valeur de tarage de la valve de régulation de pression. Ceci peut se produire en permanence en cas de fuites des chambres haute pression vers les chambres basse pression, ou lorsque l'on recharge par injection les chambres basse pression, ou encore lorsque l'on modifie pour l'augmenter la pression différentielle entre la haute et la basse pression. Le dispositif fonctionne alors comme une pompe actionnée par les mouvements du patient.

Si, en revanche, le mouvement d'inclinaison est lent, comme montré à la figure 17b, le fluide a le temps de s'écouler des chambres basse pression droite 309 à 308 sans que se produisent de fortes surpressions. Le clapet anti-retour 319G ne s'ouvrira pas.

Lorsque le patient se redresse, les pressions évoluent comme montré à la figure 17c. Etant donné que les pressions du coté droit sont, au départ, supérieures à ce qu'elles sont du coté gauche, il est peu probable qu'au cours de la durée t₃ du mouvement, la basse pression régnant dans la chambre 309 gauche devienne supérieure à la haute pression régnant dans la chambre 314 droite.

Ce n'est que lorsque le patient se penchera suffisamment vivement du coté gauche, cas représenté à la figure 17d, symétrique de celui de la figure 17a, que se rétablira à sa valeur de consigne la pression différentielle entre la haute pression droite et la basse pression gauche. On voit sur cette figure la durée t₄ du mouvement et celle t₅ pendant laquelle la basse pression gauche devient supérieure à la haute pression droite.

On comprend que ce qui vient d'être décrit étape par étape en référence aux figures 17, se produit en fait en permanence lorsque le patient bouge de façon normale en adoptant successivement différentes postures naturelles. On constate par conséquent qu'il en résulte une adaptation biomécanique continuelle des implants aux sollicitations qui lui sont imposées par le patient.

En vue du principe de l'auto-recharge expliqué en référence aux figures 17a à 17d, on voit que les pointes de pression trop aiguës vont s'aplatir du fait que le fluide est laminé lors de son écoulement dans le clapet anti-retour. Ceci réalise un amortissement additionnel lorsque la cellule est sollicitée suffisamment brusquement pour que la basse pression dépasse la haute pression. Si l'on ajoute, comme décrit plus haut, la variabilité du coefficient d'amortissement en fonction des charges, on se trouve en face d'un dispositif doué d'une intéressante capacité d'autorégulation.

Un autre avantage de ce mode de fonctionnement réside dans le fait qu'il se produit une circulation de fluide pratiquement permanente dans le circuit hydraulique de l'implant de l'invention. Cette circulation évite les risques d'encrassement et limite donc la nécessité des opérations de maintenance.

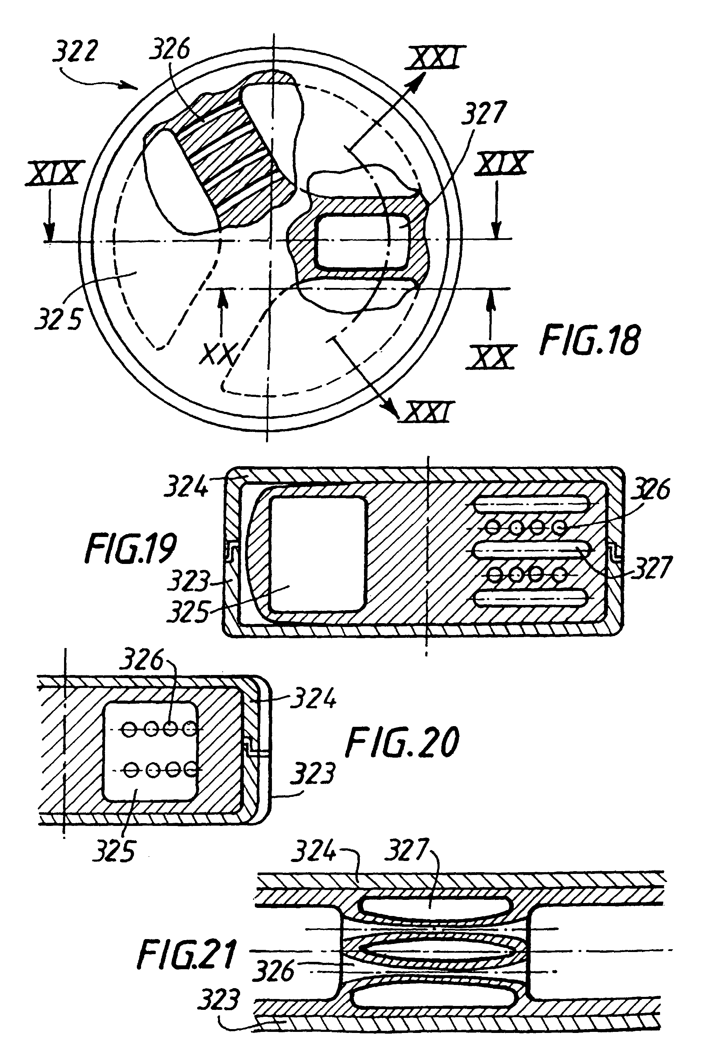

On voit sur les figures 18 à 22 un autre mode de réalisation 322 des implants 305.

L'implant 322 est toujours réalisé sous la forme d'une structure alvéolaire en élastomère incluse dans un boîtier composé d'un demi-boîtier inférieur 323 et d'un demi-boîtier supérieur 324.

La structure alvéolaire de ce mode de réalisation est en forme de disque et forme trois chambres basse pression 325 en forme de secteurs, réparties sensiblement également autour de l'axe du disque, à 120° les unes des autres. Les trois chambres 325 communiquent par un réseau de conduits calibrés 326, ici disposés en deux nappes transversales.

Entre les chambres basse pression 325 sont disposées trois groupes de chambres haute pression 327, communicant entre elles par tout moyen convenable. Les chambres 327 sont de forme aplatie et chaque groupe en compte trois, intercalées avec les nappes de conduits 326. La pression dans les chambres haute pression détermine donc la section des conduits 326 et donc leurs caractéristiques de viscosité.

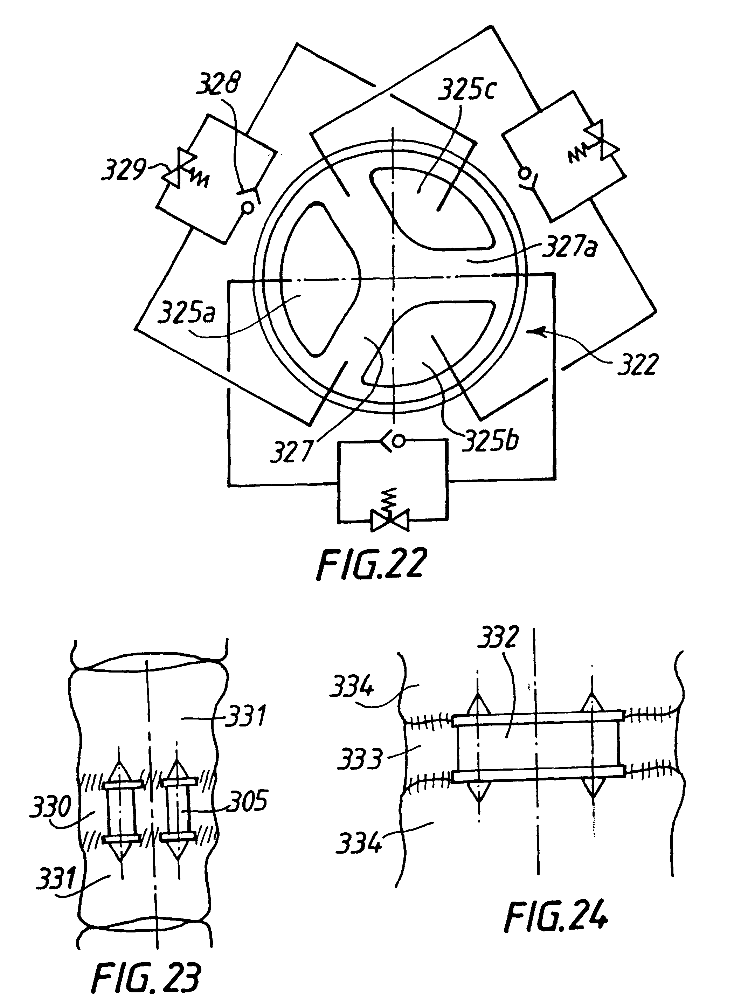

La figure 22 montre que les chambres basse pression sont reliées aux chambres haute pression par un ensemble de clapets anti-retour et de valves de régulation de pression. Chaque chambre basse pression 325 est reliée à la chambre haute pression 327 qui lui est diamétralement opposée par un clapet anti-retour 328 s'ouvrant dans le sens de la chambre 325 à la chambre 327, en parallèle avec une valve 329 de régulation de pression.

Il n'y a donc pas ici, comme dans le mode de réalisation des figures 14 et 15, et comme modélisé à la figure 17, croisement des liaisons entre les chambres basse et haute pression de deux implants montés en parallèle. En outre, il n'y a ici qu'une sorte de chambres basse pression.

Lors d'une compression axiale, le fluide contenu dans les conduits 326 est, du fait de l'épaisseur des parois de ces derniers, expulsé vers les chambres 325 avec un comportement de fluide visqueux. Les parois de ces chambres sont donc repoussées vers l'extérieur, donnant par ailleurs à l'implant son comportement élastique. De ce point de vue, cet implant se comporte comme celui du mode de réalisation précédent.

En revanche, cet implant possède un comportement particulier vis-à-vis des charges non axiales. Par exemple, si l'on imagine une charge exercée du coté gauche de la figure 22, la chambre basse pression 325a va être comprimée tandis que les chambres 325b et 325c vont se trouver en dépression. La paroi de la chambre 325a va donc s'étirer tandis qu'une partie du fluide contenu dans cette chambre va s'écouler dans les chambres 325b et 325c par les conduits 326, aspiré de plus par la dépression régnant dans ces dernières chambres.

Lorsque le mouvement est suffisamment ample et rapide, la paroi extérieure de la chambre basse pression 325a vient au contact du boîtier. Le comportement élastique de l'implant est alors stoppé, de sorte que la pression s'élève brutalement dans la chambre basse pression 325a. Cette pression peut ainsi devenir supérieure à celle régnant dans la chambre haute pression 327a qui lui fait face, engageant le processus de régulation de la pression différentielle décrit ci-dessus en référence au mode de réalisation précédent.

Un implant selon ce deuxième mode de réalisation pourrait donc être utilisé seul tout en possédant la fonction de régulation de la pression différentielle.

Divers montages peuvent être envisagés pour les implants qui viennent d'être décrits, outre celui qui a déjà été représenté et décrit en référence à la figure 13

On voit sur la figure 23 une greffe osseuse 330 disposée entre deux vertèbres 331. On a ici placé dans la greffe deux implants 305 tels que ceux des figures 14 et 15, symétriquement par rapport au plan médian du corps du patient. Les interconnexions entre les implants et les principes de fonctionnement sont les mêmes que ceux décrits en référence aux figures 14 à 17.

La figure 24 montre un implant unique 332 tel que décrit en référence aux figures 18 à 22, monté dans une greffe 333 elle-même disposée entre deux vertèbres 334. Une telle solution assure de bonnes performances vis-à-vis des flexions aussi bien latérales que frontales.

On va maintenant décrire des implants articulaires réalisés conformément à l'invention.

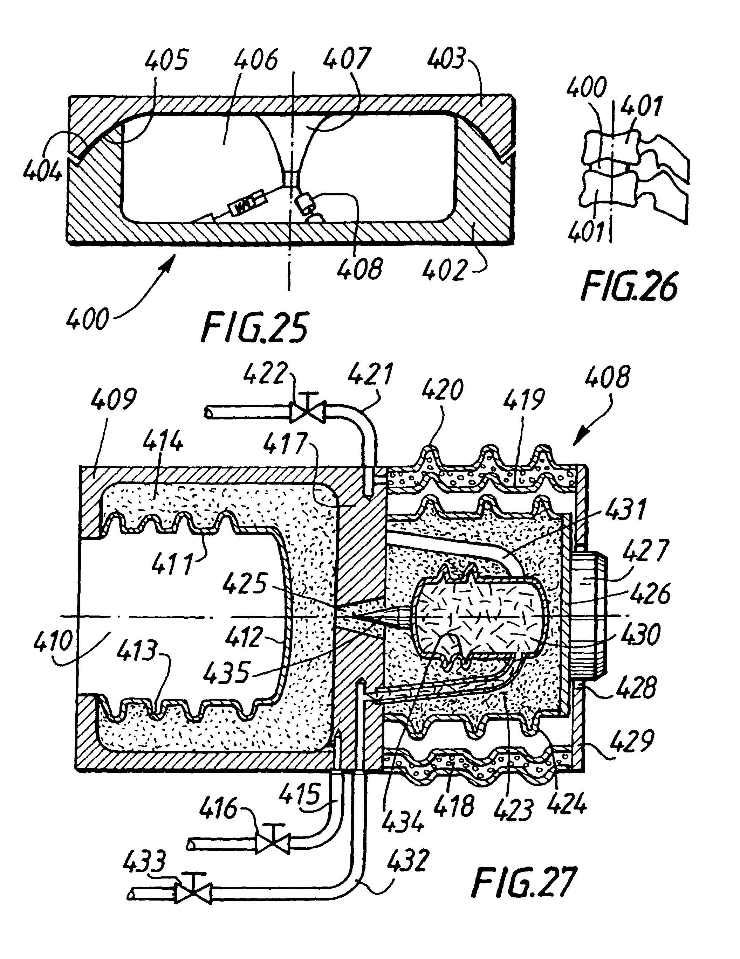

On voit à la figure 25 un implant, ou prothèse intervertébrale 400, destiné à être disposé, comme montré à la figure 26, entre deux vertèbres 401, à la place d'un disque intervertébral. Cet implant doit donc permettre certains mouvements entre les vertèbres 401, contrairement à ce qui se produit dans le cas d'une arthrodèse.

L'implant 400 est formé d'une manière générale d'un boîtier comprenant un fond 402 et un couvercle 403. Le couvercle 403 est en appui sur le fond 402 grâce à deux surfaces sphériques de même rayon, la surface 404 du fond faisant face vers le haut et la surface 405 du couvercle faisant face vers le bas. Ainsi, le couvercle 403 peut pivoter par rapport au fond 402 autour de trois axes.

Le fond 402 est creux, de manière à ménager un espace 406 à l'intérieur de l'implant. Le couvercle 403 forme une saillie 407 dans cet espace, saillie dont l'extrémité est reliée au fond par trois "ligaments" internes 408 viscoélastiques, que l'on va maintenant décrire en référence à la figure 27.

Chaque ligament 408 comprend un corps creux rigide 409, sensiblement cylindrique et possédant à une de ses extrémités une ouverture à l'air libre 410. Cette ouverture est fermée par une ampoule, ou soufflet élastique 411. Le soufflet 411 est un cylindre fermé à son extrémité opposée à l'ouverture 410 par un fond 412, et dont la paroi latérale forme un bourrelet hélicoïdal 413 à pas variable, croissant de l'ouverture 410 au fond 412.

Le corps 409 et le soufflet 411 délimitent une chambre 414 contenant un fluide hydraulique qui est alimenté et dont la pression peut être réglée à partir d'une canalisation 415 et d'une vanne 416.

A l'opposé du corps 409, le fond 417 de ce corps porte une chambre cylindrique annulaire 418 dont les parois intérieure 419 et extérieure 420 sont elles aussi constituées par des soufflets. La chambre 418 contient un fluide hydraulique qui est alimenté et dont la pression peut être réglée à partir d'une canalisation 421 et d'une vanne 422.

Au centre de la chambre annulaire 418, une autre chambre cylindrique 423 a sa paroi formée d'un soufflet 424. La chambre 423 communique avec la chambre 414 par un orifice calibré 425 percé dans le fond 417 du corps 409. La chambre 423 est donc alimentée et pressurisée à partir de la chambre 414.

L'extrémité du soufflet 424 opposée au fond 417 est fermée par une plaque 426 portant une pièce d'appui 427 traversant un orifice 428 d'une plaque d'extrémité 429 de la chambre annulaire 418. Les plaques d'extrémité 426 et 429 sont solidaires.

A l'intérieur de la chambre 423 est formée une chambre 430 montée sur le fond 417 du corps 409 à l'aide de montants 431. L'un de ces montants 431 est creux et permet d'alimenter et de pressuriser la chambre 430 en fluide hydraulique à partir d'une canalisation 432 et d'une vanne 433.

La paroi de la chambre 430 forme, entre les points de fixation des montants 431 et le fond 417, un soufflet 434. Le fond de la chambre 430 faisant face au fond 418 du corps 409 porte un pointeau 435 pénétrant dans l'orifice calibré 425.

L'implant est ancré à la saillie 407 et au fond 402 par ses éléments 409 et 427.

On comprend aisément que la longueur du ligament 408 est fonction de la pression dans la chambre annulaire 418, qui détermine l'allongement des soufflets 419 et 420. Cette longueur peut donc être ajustée à partir de la vanne 422.

Par ailleurs, le coefficient d'amortissement du ligament 408 est fonction de la section libre de l'orifice calibré 425, et donc de l'enfoncement du pointeau 435. Ce coefficient peut donc être ajusté à partir de la vanne 433.

Enfin, le soufflet peut être assimilé, en ce qui concerne son élasticité, à un ressort hélicoïdal à pas variable, qui devient de plus en plus raide au fur et à mesure qu'il est plus comprimé et que ses spires viennent progressivement en contact. Ce soufflet détermine l'élasticité du ligament 408 puisque, lorsque ce dernier est comprimé, il s'oppose élastiquement à la pénétration du fluide hydraulique dans la chambre 414 par l'orifice 425. Le coefficient d'élasticité peut donc être ajusté à partir de la vanne 416, en précomprimant plus ou moins le soufflet 411.

On observera qu'une structure similaire à celle du ligament qui vient d'être décrit, pourrait être utilisée à la place du dispositif de la figure 7, afin de rendre ses différentes fonctions réglables.

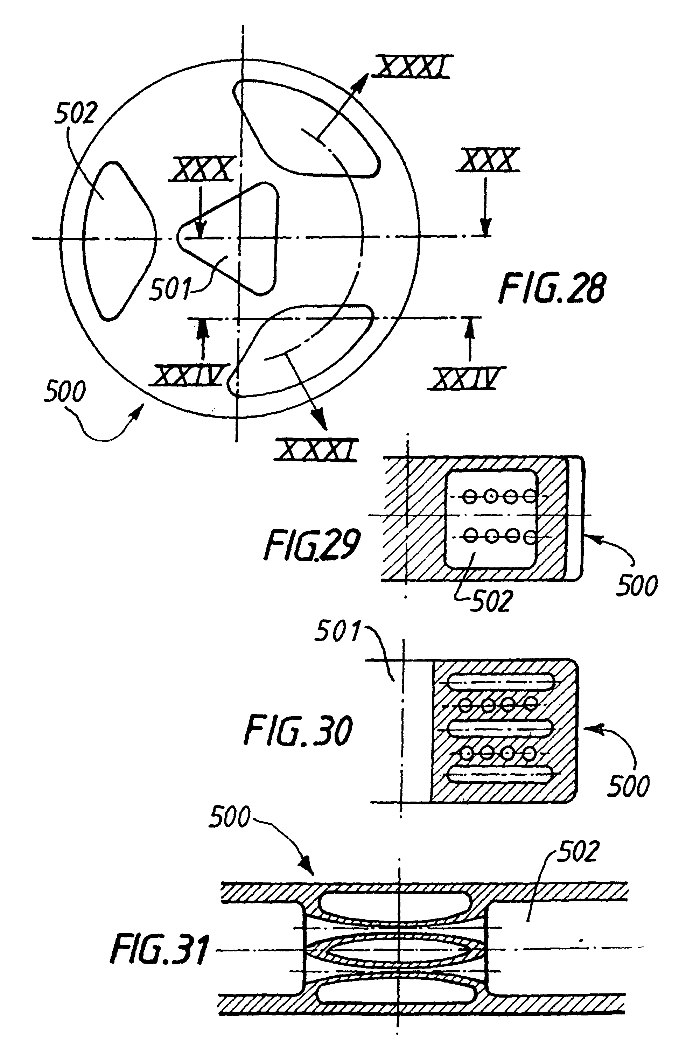

On décrira maintenant, en référence aux figures 28 à 31, une structure alvéolaire 500 en élastomère susceptible de remplacer les trois ligaments 408 de la figure 25.

Cette structure est pratiquement identique à celle de l'implant 322 des figures 18 à 21 (la figure 28 a été schématisée). On observera toutefois que la structure 500 possède ici un orifice 501 de section triangulaire pour recevoir une saillie du couvercle, similaire à la saillie 407, et destinée à former un pivot entre le fond et le couvercle de la prothèse.

Les interconnexions entre chambres sont les mêmes que dans le cas de l'implant 322, et le fonctionnement de la présente prothèse et de l'implant seront les mêmes du point de vue hydraulique.

Les différences résident dans la façon dont sont appliqués les efforts. Il s'agira ici d'efforts essentiellement transversaux appliqués aux chambres basse pression 502 par la saillie du couvercle.

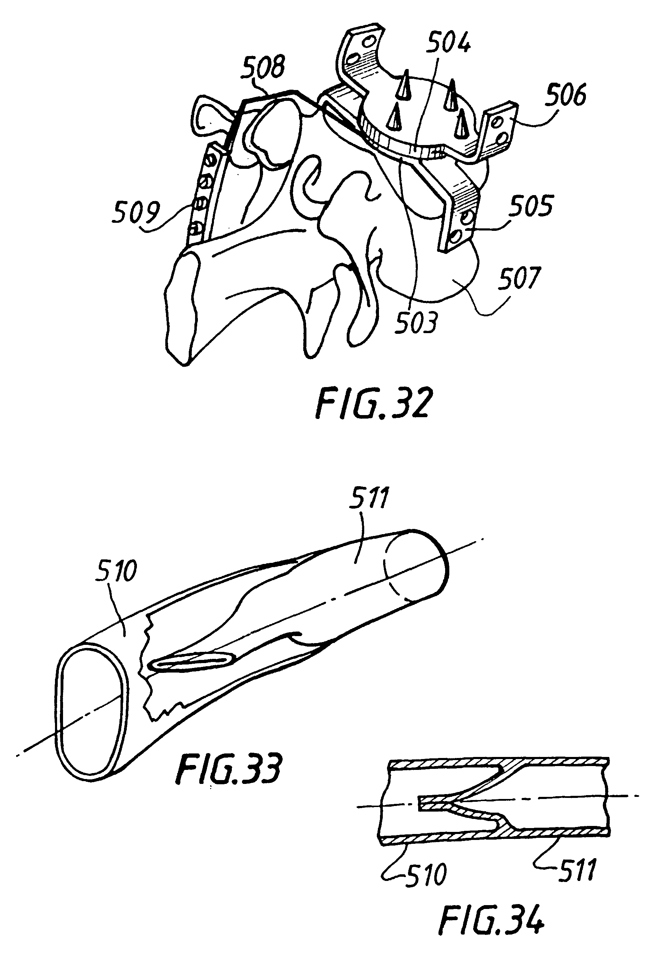

La figure 32 montre un montage possible des prothèses des figures 25 à 31.

On voit sur cette figure le fond 503 et le couvercle 504 de la prothèse. Le fond 503 est muni de ferrures 505 et le couvercle 504 de ferrures 506 pour leur fixation respectivement à une vertèbre inférieure 507 et à une vertèbre supérieure non représentée.

Les différentes chambres hydrauliques sont reliées par des canalisations 508 à un ensemble de boutons de réglage sous-cutanés 509, notamment à pression, disposés derrière les vertèbres. Des sécurités sont de préférence prévues afin d'éviter des manoeuvres intempestives des boutons. En variante, on pourrait prévoir des réglages entièrement hydraulique, à l'aide d'un site d'accès sous-cutané relié aux différentes chambres par un tiroir de distribution.

On voit sur les figures 33 et 34, respectivement en perspective partiellement arrachée et en coupe transversale, un clapet anti-retour susceptible d'être utilisé dans l'invention.

Ce clapet est composé d'un conduit 510 relié à la haute pression et d'un conduit 511 relié à la basse pression. Ces conduits sont ici coaxiaux et l'extrémité du conduit basse pression 511 est engagée à l'intérieur de l'extrémité du conduit haute pression 510. L'extrémité du conduit 511 intérieure au conduit 510 est aplatie.

Tant que la haute pression est supérieure à la basse pression, L'extrémité du conduit 511 demeure aplatie et le clapet reste donc fermé, sans possibilité d'écoulement du conduit 511 au conduit 510. Mais lorsque la basse pression devient supérieure à la haute pression, l'extrémité du conduit 511 s'ouvre et du fluide s'écoule du conduit 511 au conduit 510.

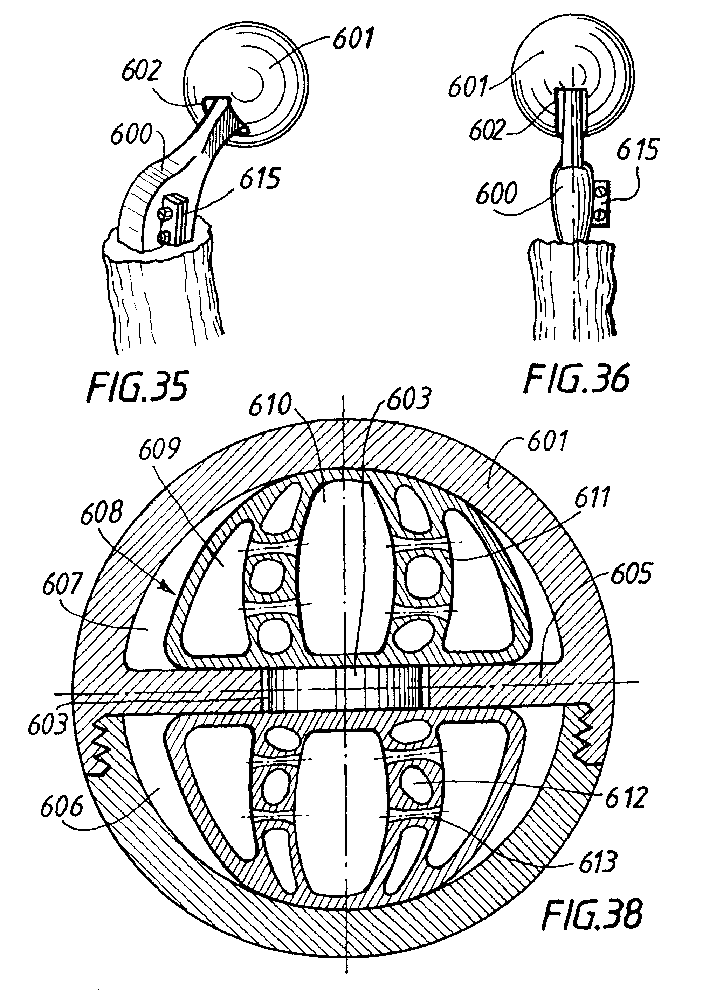

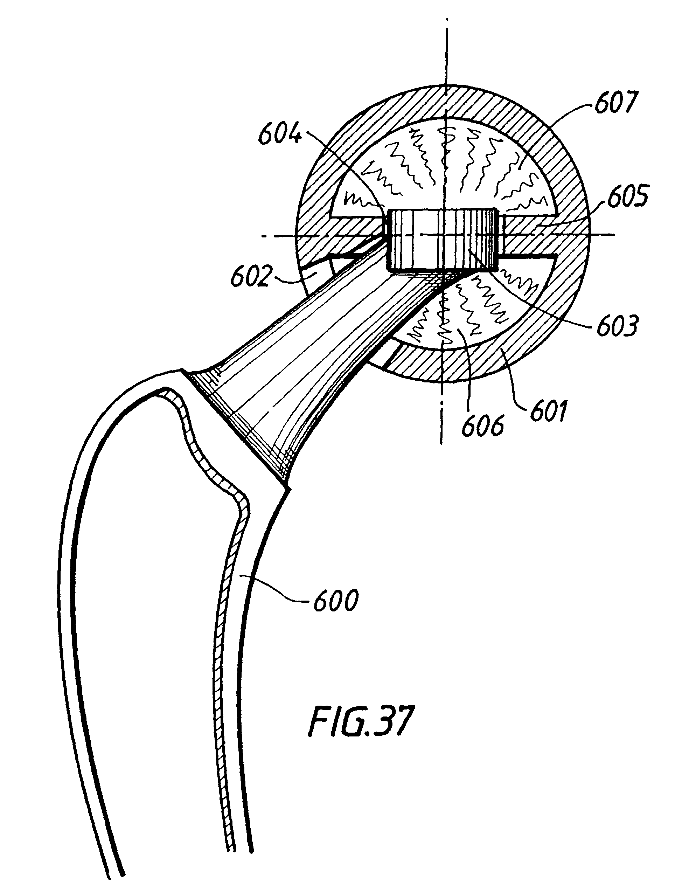

Enfin, les figures 35 à 39 illustrent une prothèse coxo-fémorale réalisée conformément aux principes de l'invention.

Cette prothèse est destinée à être utilisée après fracture du col du fémur et résection de sa partie supérieure. Elle comprend une broche 600 dont une extrémité est destinée à être fixée dans la partie conservée du fémur, et une sphère creuse 601 dont la paroi comporte un orifice 602 pour permettre sa traversée par l'autre extrémité de la broche 600.

L'extrémité supérieure de la broche 600, intérieure à la sphère 601, est solidaire d'un plateau cylindrique 603. Ce dernier est susceptible de coulisser et de former piston dans un orifice circulaire 604 d'une cloison interne 605 de la sphère. L'axe du plateau 603 et de l'orifice 604 passe sensiblement par l'autre extrémité du fémur, au niveau de l'articulation du genou.

La cloison 605 délimite à l'intérieur de la sphère, avec le piston 603, deux chambres 606 et 607, respectivement inférieure et supérieure. Les chambres 606 et 607 contiennent des dispositifs viscoélastiques qui déterminent le mouvement relatif de la broche 600 et de la sphère 601, en fonction des efforts appliqués.

Dans un mode de réalisation particulièrement simple, ces dispositifs viscoélastiques peuvent être simplement constitués d'une mousse élastique remplissant les chambres 606 et 607, et d'un orifice calibré formé dans le plateau 603. La mousse contient un fluide hydraulique, et un joint convenable est disposé au niveau de l'orifice 602.

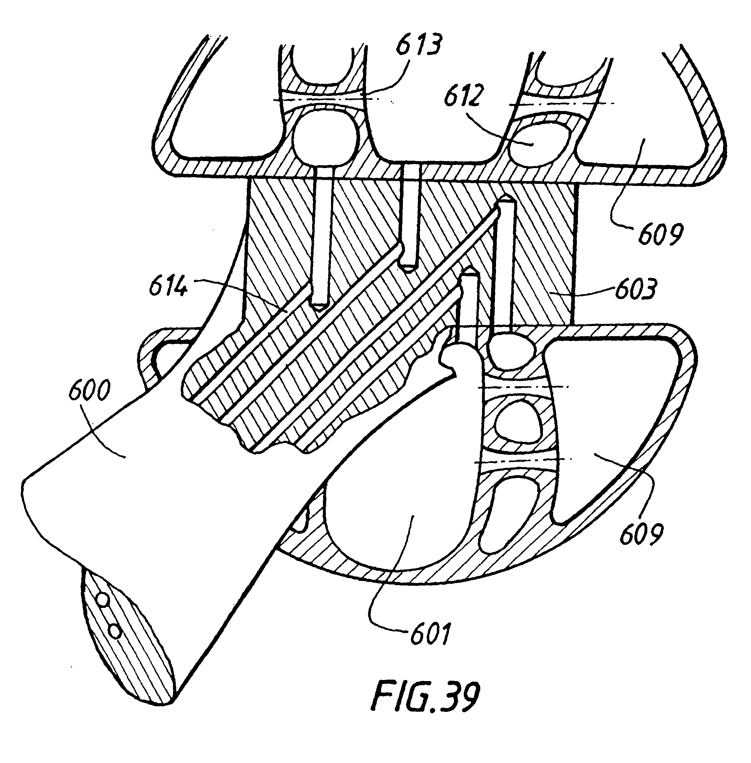

On préférera toutefois le mode de réalisation des figures 38 et 39.

Les dispositifs viscoélastiques 608 sont ici réalisés sous une forme pratiquement identique à celle des structures 307 des implants 305. La différence réside dans le fait que les structures 307 sont de forme générale cylindrique, alors que les dispositifs 608 ont une forme générale hémisphérique. Mais ils sont de la même manière composés pour l'essentiel d'une chambre basse pression périphérique 609 et d'une chambre basse pression centrale 610, séparées par une paroi 611.

Dans l'épaisseur de la paroi 611 sont formées des chambres haute pression annulaires 612, intercalées avec des conduits calibrés 613 mettant en communication les chambres basse pression 609 et 610. Les interconnexions croisées entre chambres sont réalisées comme précédemment.

Lorsque la prothèse est sollicitée, le plateau 603 comprime l'un des dispositifs 608 tandis que l'autre dispositif est mis en dépression. Tout ce qui a été indiqué à propos du fonctionnement des implants jumeaux 305 reste valable dans le cas présent.

On observera ici que, lorsqu'un dispositif 608 est comprimé, sa ligne de tangence avec la surface intérieure de la sphère se rapproche de la cloison 605. La surface de paroi extérieure élastique diminue donc, ce qui a pour effet d'augmenter la raideur élastique du dispositif.

Les interconnexions entre chambres s'effectuent comme montré à la figure 39, par des perçages 614 réalisés dans le plateau 603 et dans la broche 600. Ces perçages débouchent au niveau d'une boîte à boutons 615 (figures 35 et 36). Cette boîte est disposée de façon sous-cutanée de manière à être facilement accessible pour permettre les réglages nécessaires.

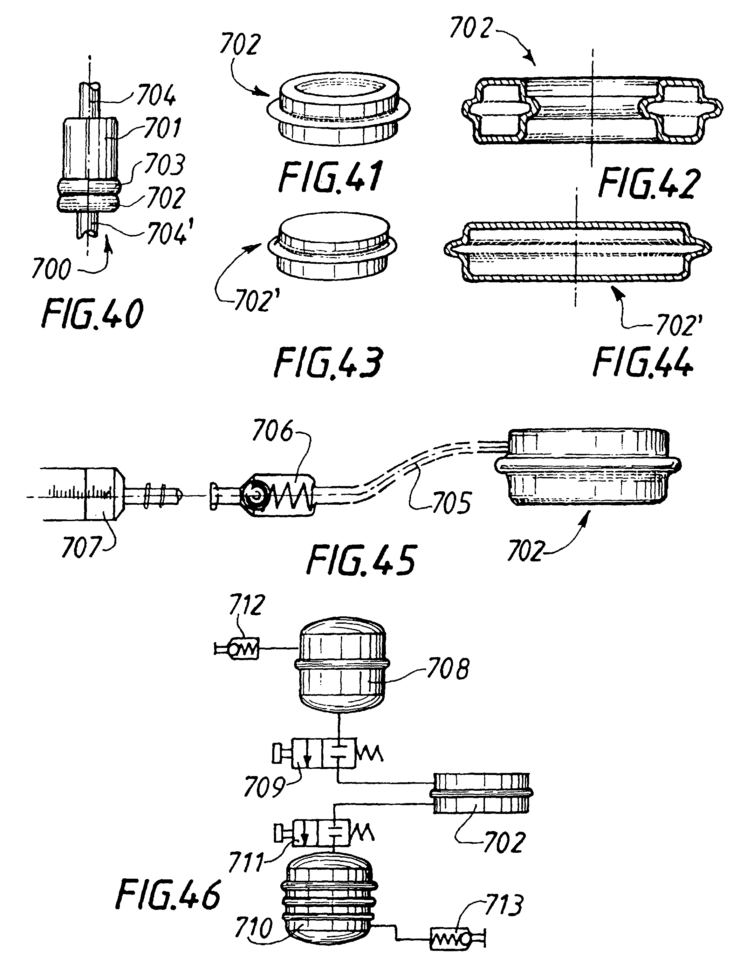

L'implant 700 de la figure 40 comprend d'une manière générale une cellule viscoélastique 701, par exemple du type de celle de l'implant 305 des figures 13 et suivantes ou du ligament 408 de la figure 27, un organe 702 de réglage de distance, ici de réglage de hauteur, et une cellule de recharge 703. Ces organes sont disposés mécaniquement en série, en appui, avec les demi broches 704, 704' de l'arthrodèse, qui peuvent appartenir aux deux montant d'un cadre. Les demi broches 704, 704' supportent par conséquent la pression du corps du patient variable en fonction de sa posture.

L'organe de réglage 702 peut être réalisé comme montré aux figures 41 et 42, sous la forme d'un soufflet torique gonflable, expansible axialement. Son espace central libre permet de loger éventuellement une protubérance de la cellule viscoélastique.

En variante, l'organe de réglage peut se présenter sous la forme du soufflet 702' en forme de disque des figures 43 et 44.

L'organe de réglage 702 (ou 702') peut être relié par un conduit 705 à une valve 706 de gonflage et de dégonflage qui peut elle-même être connectée à une pompe 707. Il est ainsi possible de régler l'épaisseur de l'organe 702. On peut donc régler non seulement la longueur totale de la prothèse, mais également l'angle formé entre sa partie supérieure (les demi broches 704) et sa partie inférieure (les demi broches 704'), par gonflage différentiel des deux organes 702 de la prothèse.

On observera que la valve 706 peut elle-même être implantée, auquel cas on y accède soit par incision cutanée, soit par l'intermédiaire d'un site d'accès, ou extérieure, le conduit 705 étant transcutané.

Dans un autre mode de réalisation, représenté à la figure 46, le gonflage des organes de réglage s'effectue à l'aide d'un réservoir haute pression 708, ici dilatable, et d'une valve à tiroir 709. De façon similaire, le dégonflage de ces organes s'effectue dans un collecteur de drainage basse pression 710, également dilatable, par l'intermédiaire d'une autre valve à tiroir 711 (ou de la même, alors à trois voies).

De plus, une valve de remplissage 712 permet de remplir le réservoir 708 et une valve de soutirage 713 permet de vider le réservoir 710. Les réservoirs 708 et 710 sont implantés et les valves 712 et 713 peuvent être externes ou implantées, comme dans le cas de la valve 706. Elles seront toutefois généralement implantées, leur accès devant être beaucoup moins fréquent que celui de la valve 706.

Les cellules de recharge 703, dont la fonction sera décrite ci-après, sont en tout point similaires aux organes de réglage 702. Toutefois, elles sont reliées aux réservoirs haute et basse pression 708 et 710 non pas par des valves à tiroir, mais par des clapets anti-retour 714 et 715 respectivement. Les clapets 714 sont connectés des cellules 703 au réservoir haute pression 708, et les clapets sont connectés du réservoir basse pression 710 aux cellules 703.

Les cellules 703 font fonction de pompe pour recharger le réservoir haute pression 708. En effet, lorsque le patient se penche, par exemple vers la droite, la cellule 703D est comprimée. Quand la pression dans cette cellule dépasse la pression dans le réservoir 708, le clapet 714D s'ouvre et du fluide passe de la cellule 703 au réservoir 708. Simultanément, la cellule gauche 703G aspire du fluide du réservoir basse pression 710.

Une valve 716 de régulation de pression empêche ici la haute pression de dépasser une valeur prédéterminée.

On remarquera que si l'on vise uniquement à des corrections angulaires, le dispositif de recharge peut être grandement simplifié, et notamment les cellules de recharges supprimées. Il suffit en effet dans ce cas de relier les organes de réglage par une valve à tiroir. Le patient se penchant par exemple à droite, la valve est ouverte de sorte que du fluide passe de droite à gauche, puis refermée. Lorsque le patient se sera redressé, la hauteur du coté gauche aura augmentée et celle du coté droit aura diminuée.

Les cellules viscoélastiques n'ont pas été décrites dans ce mode de réalisation. On notera simplement que, dans le cas où, comme par exemple les implants 305 ou les ligaments 408, elles comprennent des chambres haute pression, celles-ci peuvent être reliées au réservoir haute pression 708 par une vanne, par exemple à tiroir. Il est ainsi possible de raidir très aisément le comportement visqueux de ces cellules. Les chambres basse pression des cellules viscoélastiques peuvent d'ailleurs être, pour leur part, reliées au réservoir basse pression 710.

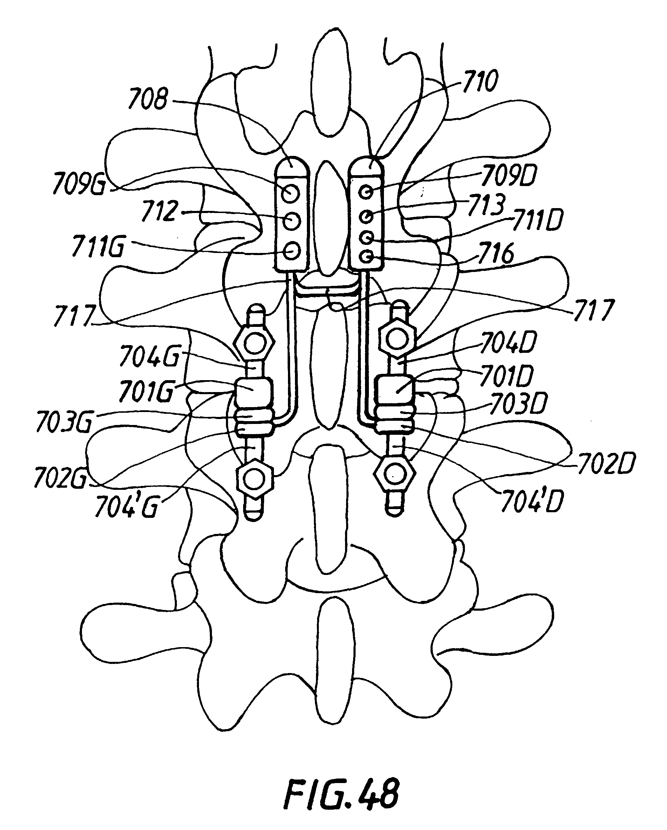

La figure 48 montre une implantation possible pour les organes qui viennent d'être décrits. Les conduits de liaison possèdent d'une manière générale la référence 717. On observera que tous ces organes peuvent être de dimensions très réduites, et les volumes hydrauliques très faibles.

Les caractéristiques physiques des implants qui viennent d'être décrits peuvent être modifiées en postopératoire par tout moyen, et notamment par des moyens totalement non invasifs, et ceci en ce qui concerne aussi bien leurs propriétés viscoélastiques, que leurs dimensions, leur inclinaison latérale ou antéro-postérieure, ou leur résistance opposée à la rotation.

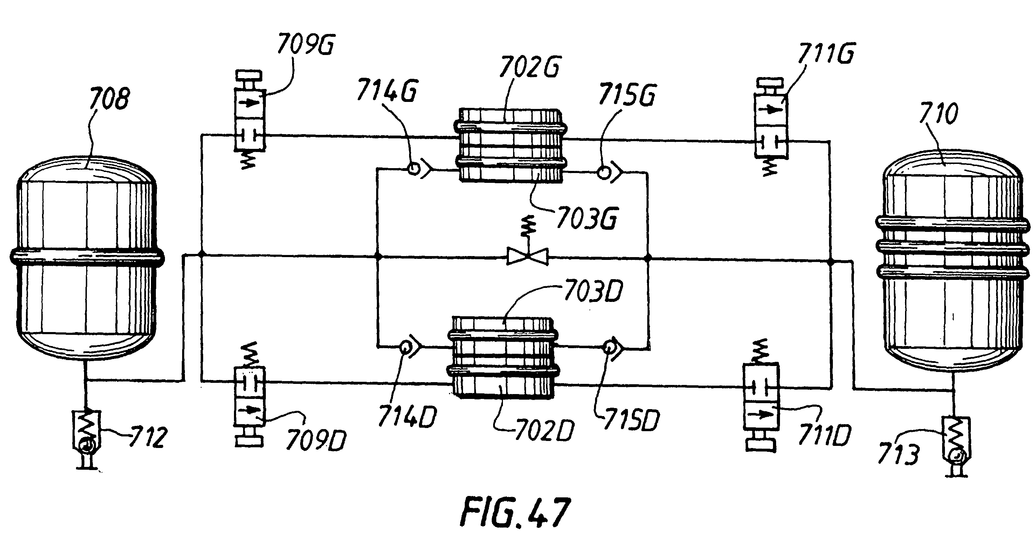

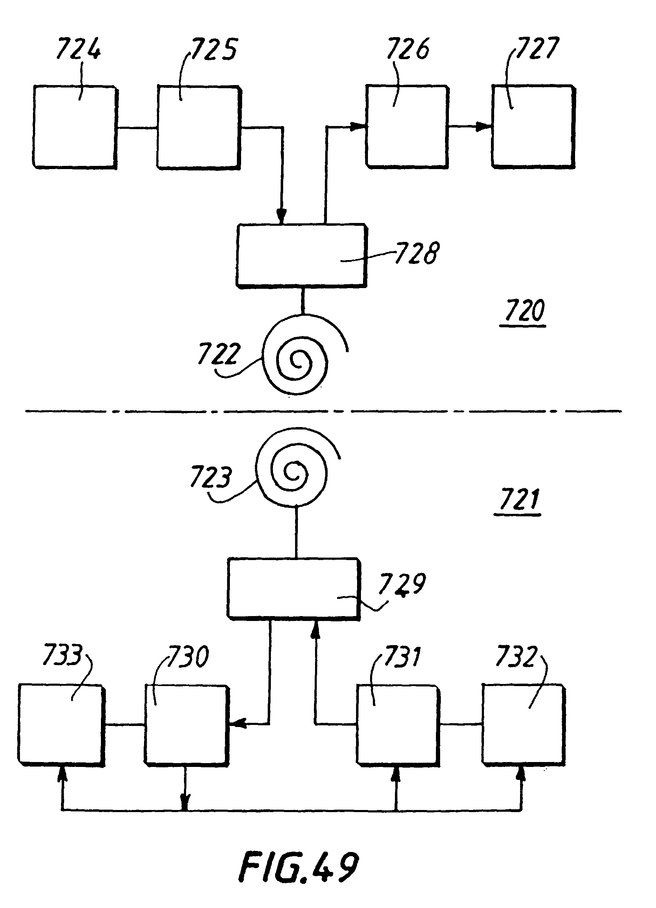

A titre d'exemple, la figure 49 illustre un circuit électronique de commande pour un implant du type de celui représenté à la figure 47.

Ce circuit est réalisé en deux parties, une partie extracorporelle 720 et une partie implantée 721 Ces deux parties sont en relation par l'intermédiaire de deux antennes 722 et 723 respectivement.

La partie de circuit 720 comprend une alimentation 724, une télécommande 725, un module de détection et d'amplification 726, et un moniteur 727. La télécommande 725 attaque un multiplexeur radiofréquence 728 connecté à l'antenne 722.

La partie de circuit 721 comprend également un multiplexeur radiofréquence 729 connecté à l'antenne 723. Le multiplexeur 729 est relié à un convertisseur radiofréquence/tension continue 730 qui assure l'alimentation électrique des autres modules de la partie de circuit 721, à savoir un oscillateur haute fréquence 731 et des capteurs 732, ainsi que d'actionneurs 733.

Dans le cas des figures 40 et 47 , les capteurs 732 peuvent notamment être des capteurs de pression dans les organes 702 de réglage de distance, ainsi éventuellement que dans les chambres des cellules viscoélastiques 701. Les actionneurs peuvent comprendre des électrovannes telles que les valves à tiroir 709 et 711. Plus particulièrement, les capteurs de pression peuvent comprendre, pour chaque côté du patient, un capteur pour chaque chambre basse pression et un capteur pour la chambre haute pression. On peut également prévoir des capteurs de pression sur les moyens de fixation, tels que vis ou crochets, des implants ainsi que sur l'os éventuellement ponté.

Lorsque le médecin souhaite connaître et éventuellement régler la pression dans, par exemple, les organes 702, il actionne la télécommande 725. L'antenne 722, placée à proximité de l'antenne 723 émet un code détecté et exploité par la partie implantée 721. Par ailleurs, la radiofréquence est transformée en tension continue d'alimentation des capteurs 732, de l'oscillateur 731, et du multiplexeur 729. La partie implantée émet alors à son tour un code contenant l'information de pression, qui est détectée et affichée sur le moniteur 727. Le réglage des organes 702 à l'aide des électrovannes 733 s'effectue de la même manière.

On observera que l'on peut réaliser, grâce à l'invention, une étude très fine du comportement des implants. Dans le cas, par exemple, d'un implant vertébral, on peut relever sa réponse en fréquence, ou sa réponse impulsionnelle, en faisant asseoir le patient sur un siège muni de moyens moteurs pouvant agir dans toutes les directions souhaitées, et en enregistrant la réponse des capteurs de pression. Le réglage des différentes pressions à l'arrêt peut ainsi être déterminé avec une grande précision.

On notera qu'un implant selon l'invention peut inclure des moyens de neuro-stimulation analgésique de type connu, ainsi qu'une pompe programmable de délivrance médicamenteuse.

D'une manière générale, pour tous les implants décrits ci-dessus et ne possédant pas de télécommande par radiofréquences ou autre, on prévoira des dispositifs de réglage accessibles soit directement, s'il s'agit de boutons sous-cutanés, soit moyennant une intervention bénigne. Les moyens de réglage par télécommande sans contact physique peuvent être des vannes rotatives du type "robinet", à plusieurs voies, dont la rotation est provoquée par un champ magnétique rotatif extérieur. Un ressort spiral rétablit l'équilibre en position fermée. Il est en effet intéressant de pouvoir effectuer les réglages souhaités relativement souvent, soit de manière planifiée, par exemple pour diminuer progressivement le coefficient d'amortissement au fur et à mesure de la consolidation d'une greffe, soit ponctuellement, par exemple pour soulager une douleur.

De même, tous les implants ci-dessus peuvent être pourvus de perfectionnements qui n'ont été décrits qu'en référence à certains des modes de réalisation. Il en est par exemple ainsi de l'adaptation dimensionnelle prévue dans les implants des figures 14 et 27. Une telle disposition est notamment utile sur tout implant destiné à un enfant, appelé à grandir, ou à une personne âgée dont la taille décroît peu à peu.

## Revendications

1. Implant squelettique destiné à assurer une liaison entre au moins deux éléments (4,5) du squelette, ledit implant étant réalisé en au moins deux parties (7,8), chacune étant susceptible d'être reliée à un desdits éléments, **caractérisé en ce qu'**il comprend entre lesdites deux parties, des moyens amortisseurs (9) à coefficient d'amortissement variable, et/ou des moyens de réglage de la distance entre lesdites deux parties, lesdits moyens d'amortissement et/ou lesdits moyens de réglage comprenant un ou des organes déformables contenant un fluide hydraulique, ledit implant comprenant, en outre, des moyens de recharge de fluide et sensibles à des mouvements corporels pour adresser du fluide sous pression auxdits organes.

2. Implant selon la revendication 1 **caractérisé en ce que** lesdits moyens de recharge (703) sont disposés en série entre lesdites deux parties (704, 704) pour être actionnés par les déplacements corporels desdites parties.

3. Implant selon l'une quelconque des revendications 1 et 2, comprenant des moyens (241) de limitation de la course du dispositif amortisseur.

4. Implant selon la revendication 3, dans lequel lesdits moyens, de limitation de la course sont réglables.

5. Implant selon l'une quelconque des revendications 1 à 4, réalisé sous la forme d'un organe allongé tel qu'une vis, comprenant deux parties d'extrémités (105, 106) reliées par une partie médiane souple (107), la partie médiane comprenant deux chambres (111, 112)remplies de fluide hydraulique, situées de part et d'autre d'une fibre neutre et agencées l'une pour augmenter de volume, et l'autre pour diminuer de volume lorsque l'implant fléchit, lesdites chambres étant reliées par au moins un conduit calibré (113, 114).

6. Implant selon la revendication 5, dans lequel lesdites parties d'extrémité sont reliées par une paroi élastique (110) délimitant lesdites chambres avec un soufflet périphérique.

7. Implant selon l'une quelconque des revendications 5 et 6, dans lequel ledit conduit calibré est réalisé sous la forme de perçages dans au moins une des parties d'extrémité.

8. Implant selon l'une quelconque des revendications 5 à 7, comprenant une vanne (116) sur ledit conduit calibré.

9. Implant selon l'une quelconque des revendications 1 à 4, dans lequel le dispositif amortisseur comprend au moins une chambre (220) formée de deux demi-chambres réunies par un soufflet (222), chacune des demi-chambres étant reliée à une des parties de l'implant, ladite chambre étant remplie d'un fluide hydraulique, et au moins un orifice calibré (229) étant prévu dans une paroi de ladite chambre pour faire communiquer ladite chambre avec une autre chambre (221).

10. Implant selon la revendication 9, dans lequel lesdites demi-chambres sont en forme de coupelles (224, 225) dont les ouvertures se font face.

11. Implant selon l'une quelconque des revendications 9 et 10, comprenant un organe de blocage (243) allongé, amovible, engagé dans un pli dudit soufflet pour empêcher son écrasement.

12. Implant selon l'une quelconque des revendications 9 à 11, comprenant une couronne de butée (241) vissée sur une desdites demi-chambres et agencée pour coopérer avec un épaulement (242) de l'autre demi-chambre pour s'opposer au rapprochement des deux demi-chambres au delà d'une certaine limite.

13. Implant selon l'une quelconque des revendications 9 à 12, dans lequel ladite autre chambre est logée dans une jupe d'appui (230) montée sur une desdites demi-chambre autour de l'orifice calibré, à l'opposé de l'autre demi-chambre.

14. Implant selon l'une quelconque des revendications 9 à 13, dans lequel ladite autre chambre est également formée de deux demi-chambres réunies par un soufflet (223).

15. Implant selon l'une quelconque des revendications 1 à 4, comprenant en outre des moyens élastiques entre lesdites deux parties.

16. Implant selon la revendication 15, dans lequel le coefficient d'élasticité est réglable.

17. Implant selon l'une quelconque des revendication 15 et 16, comprenant deux chambres remplies de fluide hydraulique et réunies par un orifice calibré, l'une au moins des chambres contenant une ampoule de compression (411) à pression ambiante à parois élastiques.

18. Implant selon la revendication 17, dans lequel la paroi de ladite ampoule possède une élasticité progressive, et l'une desdites chambres est susceptible d'être reliée à une source (415, 416) de fluide sous pression.

19. Implant selon l'une quelconque des revendications 5 à 18, comprenant des moyens (430-435) pour régler la section dudit orifice calibré.

20. Implant selon la revendication 19, dans lequel lesdits moyens de réglage de la section de l'orifice calibré comprennent une valve à pointeau à commande hydraulique.

21. Implant selon l'une quelconque des revendications 1 à 4, dans lequel le dispositif amortisseur comprend au moins deux chambres basse pression (308, 309) dont les parois sont réalisées en matériau élastique, lesdites chambres étant reliées par au moins un conduit calibré (313) et remplies de fluide hydraulique, et agencées pour subir une variation de pression différentielle lors d'un mouvement relatif desdits éléments.

22. Implant selon la revendication 21, dans lequel une des chambres basse pression possède une paroi (312) d'élasticité relativement faible par rapport à l'élasticité des parois (311) de l'autre chambre.

23. Implant selon l'une quelconque des revendications 21 et 22, dans lequel ledit conduit calibré a sa section déterminée par la pression régnant dans une chambre haute pression (314) susceptible de comprimer ce conduit.

24. Implant selon la revendication 23, dans lequel une chambre haute pression et une chambre basse pression sont reliées, dans le sens de la basse à la haute pression, par un clapet anti-retour (319).

25. Implant selon la revendication 24, dans lequel ledit clapet anti-retour comprend un tuyau souple (511) relié à la chambre basse pression, dont une extrémité libre est engagée dans une extrémité libre d'un tuyau (510) relié à la chambre haute pression.

26. Implant selon l'une quelconque des revendications 24 et 25, dans lequel lesdites chambres sont reliées en outre reliées par une valve (320) de régulation de pression en parallèle sur le clapet anti-retour.

27. Implant selon l'ensemble de la revendication 22 et de l'une quelconque des revendications 25 à 26, dans lequel ledit clapet anti-retour est disposé entre la chambre basse pression à rigidité élevée et la chambre haute pression.

28. Implant selon l'une quelconque des revendications 21 à 27, comprenant une première chambre basse pression annulaire (308), et une deuxième chambre de révolution (309) au centre de la première chambre, lesdits conduits calibrés étant formés radialement dans la paroi (310) séparant les deux chambres.

29. Implant selon la revendication 28, dans lequel la paroi extérieure de la première chambre basse pression est relativement mince, et la paroi séparant les deux chambres basse pression est relativement épaisse.

30. Implant selon l'une quelconque des revendications 28 et 29, comprenant au moins une chambre haute pression annulaire (314) formée dans l'épaisseur de la paroi séparant les deux chambres basse pression, et agencée pour comprimer lesdits conduits calibrés.

31. Implant selon l'une quelconque des revendications 21 à 29, dans lequel ledit conduit est au moins partiellement réalisé sous la forme d'un tube en matériau élastique entouré par un tube sensiblement plus rigide, lesdits tubes étant reliés annulairement à leurs extrémités, le volume compris entre les deux tubes formant une chambre haute pression.

32. Implant selon l'une quelconque des revendications 23 à 27, sensiblement en forme de disque, comportant une pluralité de chambres basse pression (325) en forme de secteurs, reliées par lesdits conduits calibrés (326), eux-mêmes alternés dans l'épaisseur du disque avec lesdites chambres haute pression (327).

33. Implant selon l'une quelconque des revendications 1 à 32, comprenant des moyens de réglage (317) de la distance à laquelle il relie lesdits éléments.

34. Implant selon la revendication 33, dans lequel lesdits moyens de réglage comprennent un soufflet (315) agencé pour recevoir un fluide hydraulique, et des moyens de connexion dudit soufflet à une source de fluide sous pression.

35. Paire d'implants selon l'une quelconque des revendications 24 à 27, la chambre basse pression de chacun des implants étant reliée par ledit clapet anti-retour à la chambre haute pression de l'autre implant.

36. Implant selon l'une quelconque des revendications 1 à 34, dans lequel chacune desdites parties (402, 403) est articulée sur l'autre.

37. Implant selon la revendication 36, dans lequel lesdites parties possèdent des surfaces complémentaires (404, 405) en appui l'une sur l'autre, formant une articulation à rotule.

38. Implant selon la revendication 36, comprenant un pivot (407) solidaire d'un desdites parties et logé dans un espace formé entre une pluralité de chambres basse pression (502) en forme de secteurs, solidaires de l'autre partie de l'implant et reliées par lesdits conduits calibrés, eux-mêmes alternés avec des chambres haute pression.

39. Implant coxo-fémoral selon la revendication 36, comprenant une sphère creuse d'articulation (601), dont la paroi est ouverte pour permettre le passage de l'extrémité d'un corps de fixation (600), ledit dispositif amortisseur étant disposés dans ladite sphère entre la paroi de cette dernière et l'extrémité du corps de fixation.

40. Implant coxo-fémoral selon la revendication 39, dans lequel ledit dispositif amortisseur comprend un organe d'extrémité du corps de fixation agencé pour coulisser dans une découpe d'une cloison interne à la sphère, ladite cloison délimitant deux chambres dans la sphère, et au moins un orifice calibré étant formé dans ledit organe d'extrémité entre les deux dites chambres.

41. Implant coxo-fémoral selon la revendication 39, dans lequel ledit dispositif amortisseur comprend un organe d'extrémité (603) du corps de fixation disposé entre deux organes d'amortissement (608) comprenant chacun au moins deux chambres basse pression (609, 610) dont les parois sont réalisées en matériau élastique, lesdites chambres étant reliées par au moins un conduit calibré (613) et remplies de fluide hydraulique, et agencées pour subir une variation de pression différentielle lors d'un mouvement relatif de la sphère et du corps de fixation.

42. Implant selon l'une quelconque des revendications 1 à 41, dans lequel le dispositif amortisseur comprend au moins deux chambres, lesdites chambres étant reliées par au moins un conduit calibré (313; 425) et remplies de fluide hydraulique, et agencées pour subir une variation de pression différentielle lors d'un mouvement relatif desdits éléments, ledit conduit calibré ayant sa section déterminée par la pression régnant dans une chambre haute pression (314; 434).

43. Paire d'implants selon l'une quelconque des revendications 1 à 42, utilisée notamment dans le cadre d'une arthrodèse de la colonne vertébrale, chacun des implants étant associé mécaniquement en série à une broche de liaison (704, 704') de type connu.

44. Paire d'implants selon la revendication 43, dans laquelle chacun des implants comprend des moyens de réglage (702) de la distance à laquelle il relie lesdits éléments.

45. Paire d'implants selon la revendication 44, dans laquelle lesdits moyens de réglage comprennent, pour chaque implant, un organe gonflable tel qu'un soufflet, agencé pour recevoir un fluide hydraulique, et des moyens de connexion (705, 706; 709) dudit soufflet à une source de fluide sous pression.

46. Paire d'implants selon la revendication 45, dans laquelle ladite source de fluide sous pression est un réservoir (708) de fluide haute pression.

47. Paire d'implants selon la revendication 46, dans laquelle ledit réservoir haute pression est commun aux deux implants, chaque organe gonflable est également connecté à un réservoir basse pression (710), et une cellule de recharge gonflable (703) est associée mécaniquement en série à chaque broche, chaque cellule de recharge étant connectée aux réservoirs haute et basse pression par deux clapets anti-retour, l'un (715) permettant un écoulement de fluide du réservoir basse pression à la cellule de recharge, et l'autre (714) permettant un écoulement de fluide de la cellule de recharge au réservoir haute pression.

48. Paire d'implants selon la revendication 47, formée d'implants selon la revendication 2, et dans laquelle les chambres haute pression des implants sont connectées au réservoir haute pression par une vanne commandable.

49. Implant squelettique selon l'une quelconque des revendications 1 à 42, comportant des capteurs de grandeurs physiques (732), notamment de pression, alimentés électriquement et commandés extracorporellement de manière non invasive, et agencés pour transmettre leurs informations à des moyens d'affichage (727).

50. implant squelettique selon la revendication 49, comportant des actionneurs de réglage (733) également alimentés électriquement et commandés extracorporellement de manière non invasive.

51. Implant selon l'une quelconque des revendications précédentes, comportant des moyens (319) d'auto-adaptation aux mouvements du porteur des implants.

## Patentansprüche

1. Skelettimplantat zur Herstellung einer Verbindung zwischen mindestens zwei Bestandteilen des Skelettes (4,5), welches Implantat aus mindestens zwei Teilen (7,8) besteht, von denen jedes an einem der genannten Skelettbestandteile angeschlossen werden kann, **dadurch gekennzeichnet dass** es ,zwischen diesen zwei Teilen, eine Dämpfungsvorrichtung (9) deren Dämpfungskoeffizient veränderlich ist und\oder eine Vorrichtung die die Entfernung zwischen diesen zwei Teilen zu ändern erlaubt umfasst, welche Dämpfungsvorrichtung und/oder Entfernungsänderungsvorrichtung ein oder mehrere eine Flüssigkeit enthaltendes formveränderliches Organ enthält, wobei das Implantat zusätzlich eine Vorrichtung zur Wiederauffüllung dieser Flüssigkeit enthält, die auf körpereigene Bewegungen anspricht um Flüssigkeit diesem Organ unter Druck zuzuführen.

2. Implantat gemäss Anspruch 1 **dadurch gekennzeichnet, dass** die Vorrichtungen zur Wiederauffülung (703) zwischen den beiden Teilen (704,705) in Serie und so zurechtgelegt sind dass sie durch die körperlichen Bewegungen betätigt werden.

3. Implantat gemäss einer der Ansprüche 1 und 2, das Mittel (241) enthält die es gestatten die Ausdehnung der Dämpfungsvorrichtung zu begrenzen.

4. Implantat gemäss Anspruch 3, in welchem die Ausdehnungbegrenzungmittel einstellbar sind.

5. Implantat gemäss einen der Ansprüche 1 bis 4, hergestellt in Form einer ausgestreckten Vorrichtung, wie etwa einer Schraube, welche zwei durch ein biegsames Mittelteil (107) verbundene Endteile (105,106) enthält welches Mittelteil zwei Hohlräume (111,112) beträgt die mit Flüssigkeit ausgefüllt sind und an beiden Seiten einer neutrale Wand (110) so angebracht sind dass bei einer Beugung des Implantates einer der Hohlräume im Volumen zunimmt während der andere abnimmt, wobei beide Hohlräume durch mindestens ein kalibriertes Rohr verbunden sind

6. Implantat gemäss Anspruch 5, in welchem die genannten Endteile durch eine elastische Wandung (110) verbunden sind welche mit einem umhüllenden Balg beide Hohlräume bestimmt.

7. Implantat gemäss einen der Ansprüche 5 und 6, in welchem dieses kalibrierte Rohr durch Durchbohrung in mindestens einer der Endteile durchgeführt ist.

8. Implantat gemäss einer der Ansprüche 5 bis 7, welches ein Ventil an dem kalibrierten Rohr besitzt.

9. Implantat gemäss einen der Ansprüche 1 bis 4, in welchem die Dämpfungsvorrichtung zumindest einen Hohlraum (220) enthält welches aus zwei durch einen Balg (222) verbundene Halbhohlräume besteht, wobei jeder Halbhohlraum mit einem Teil des Implantates verbunden ist und der Hohlraum mit Flüssigkeit gefüllt ist und wobei mindestens ein kalibriertes Rohr (229) in der Wand des Hohlraumes vorgesehen ist um diesen Raum mit einem anderen Raum (221)zu verbinden.

10. Implantat gemäss Anspruch 9, in welchem die Halbhohlräume kuppelförmig sind , wobei ihre offene Seiten gegenüber aufmünden.

11. Implantat gemäss einen der Ansprüche 9 und 10, welches eine ausgedehnte abnehmbare Riegelsvorrichtung(243) enthält, die in einer Falte des Balges untergebracht ist um dessen Zerdrückung zu verhindern.

12. Implantat gemäss einen der Ansprüche 9 bis 11, welches einen kranzartigen Anschlag (241) enthält der auf einen der Halbhohlräume angeschraubt ist und so auf ein entsprechendes Kranzteil (242) des anderen Halbhohlraumes einwirkt, dass die Annäherung der beiden Hohlräume unter einen Grenzwert verhindert werden kann.

13. Implantat gemäss einen der Ansprüche 9 bis 12, in welchem der andere Hohlraum sich innerhalb einem Stutszylinder (230) befindet der auf einem der Halbhohlräume um das kalibrierte Rohr herum und gegenüber des anderen Halbhohlraumes,. angebracht ist

14. Implantat gemäss einen der Ansprüche 9 bis 13, in welchem der genannte Hohlraum ebenfalls aus zwei durch einen Balg verbundene Halbhohlraümen besteht.

15. Implantat gemäss einen der Ansprüche 1 bis 4, welches ausserdem noch elastische Mittel zwischen den beiden Endteilen aufweist.

16. Implantat gemäss Anspruch 15, in welchem der Elastizitätskoeffizient verstellbar ist.

17. Implantat gemäss einen der Ansprüche 15 und 16, welches zwei mit Flüssigkeit gefüllte und durch ein kalibriertes Rohr verbundene Hohlräume besitzt, wobei zumindest einer der Hohlräume einen druckempfindlichen Behälter (411) mit elastischen Wänden aufweist, welche dem herumbestehenden Druck ausgesetzt sind.

18. Implantat gemäss Anspruch 17, in welchem zumindest eine Wand des Behälters(411) eine progressive Elastizität besitzt und einer der genannten Hohlräume mit einer Druckflüssigkeitquelle (415,416) verbunden werden kann.

19. Implantat gemäss einer der Anspruche 5 bis 18, welches eine den Durchmesser des Rohres regulierende Vorrichtung (430bis, 435) enthält die.

20. Implantat gemäss Anspruch 19, in welchem die Einstellungsmittel des kalibrierten Rohres ein hydraulisch gesteuertes Spitzventil enthalten

21. Implantat gemäss einen der Ansprüche 1 bis 4, in welchem die Dämpfungsvorrichtung mindestens zwei Niederdruckhohlräume enthält (308,309) deren Wände elastisch sind und welche Hohlräume durch mindestens ein kalibriertes Rohr (313) verbunden sind und mit Flüssigkeit gefüllt sind, so dass sie bei relativer Bewegung der Elemente einem Differenzialdruck ausgesetzt sind.

22. Implantat gemäss Anspruch 21, in welchem einer der Niederdruckhohlräume eine relativ schwach elastische Wand aufweist (312) im Vergleich zur Elastizität der Wände (311) des anderen Hohlraumes.

23. Implantat gemäss einer der Ansprüche 21 und 22, in welchem der Durchfluss durch das kalibrierte Rohr durch den Druck des Hochdruckhohlraumes (314) reguliert wird welcher Hochdruck das elastische Rohr zusammendrückt.

24. Implantat gemäss Anspruch 23, in welchem ein Hochdruckholraum und ein Niederdruckhohlraum in Richtung Tiefdruck zu Hochdruck mittels einen Rückschlagventil (319).verbunden sind

25. Implantat gemäss Anspruch 24, in welchem das Rückschlagventil einen biegsamen mit dem Niederdruck verbundenen Schlauch (511) enthält und dessen eines freie Ende in ein freies Ende eines mit dem Hochdruckraum verbundenen Schlauches (510) mündet.

26. Implantat gemäss einen der Ansprüche 24 und 25, in welchem die beiden Hohlraüme zusätzlich durch ein Druckeinstellungsventil (320) parallel zu dem Rückschlagventil verbunden sind.

27. Implantat gemäss Anspruch 22, zusammen mit einer der Ansprüche 25 und 26 , in welchem das Rückschlagventil zwischen dem steiferen Niederdruckraum und dem Hochdruckraum angelegt ist.

28. Implantat gemäss einen der Ansprüche 21 bis 27, welches einen ersten ringförmigen Niederdruckraum (308), sowie einen zweiten Hohlraum (309) im Zentrum des ersten enthält wobei die kalibrierten Röhre radial in der die beider Hohlraume trennenden Wand (311) angebracht sind.

29. Implantat gemäss Anspruch 28, in welchem die äussere Wand des ersten Niederdruckhohlraumes relativ dünn ist wobei die die beiden Tiefdruckhohlräume trennende Wand relativ dick ist.

30. Implantat gemäss einen der Ansprüche 28 und 29, welches mindestens einen in der dicken Scheidewand eingebauten, die beiden Niederdruckhohlräume trennenden. ringförmigen Hochdruckhohlraum (314 ) enthält

31. Implantat gemäss der Ansprüche 21 bis 29, in welchem das Rohr zumindest teilweise aus einem elastischem Material gebildet ist und selbst mit einem bedeutend steiferen Rohr umgeben ist, wobei beide Rohre ringförmig an ihren Enden verbunden sind und das zwischen beiden Rohren gebildete Volumen eine Hochdruckkammer bildet.

32. Implantat gemäss einen der Ansprüche 23 bis 27, mit einer scheibenartigen Form, welche mehrere kreisausschnittförmige Niederdruckkammer ( 325 ) enthält, die durch die genannten kalibrierten Rohre ( 326 ) verbunden sind, welche ihrerseits in der Scheibendicke in Wechselfolge mit den Hochdruckhohlräumen (327) eingelegt sind.

33. Implantat gemäss einer der Ansprüche 1 bis 32, welches Mittel (317) zur Einstellung der Entfernung zwischen den durch ihn verbundenen Elemente aufweist.

34. Implantat gemäss Anspruch 33, in welchem diese Einstellungsmittel einen eine Flüssigkeit empfangende Balg ( 315 )enthalten, sowie Mittel, um diesen Balg mit einer Druckflüssigkeitsquelle zu verbinden.

35. Implantatpaar gemäss einen der Ansprüche 24 bis 27, in welchem die Niederdruckkammer jedes Implantates durch ein Rückschlagventil mit der Hochdruckkammer des anderen Implantates verbunden ist.

36. Implantat gemäss einen der Ansprüche 1 bis 34, in welchem die genannten Teile ( 404, 405 ) einander angegliedert sind.

37. Implantat gemäss Anspruch 36, in welchem die genannten Teile anpassende ein Drehgelenk bildende Oberflächen besitzen ( 404, 405 )

38. Implantat gemäss Anspruch 36, mit einem an einem der genannten Teile befestigten Drehpunkt (407), welcher in einem Raum untergebracht ist, der sich zwischen einer Mehrzahl an dem anderen Teil des Implantates befestigten, kreisausschnittförmigen Niederdruckkammern ( 502 ) befindet, welche durch die genannten kalibrierten Rohre in einer Wechselfolge mit den Hochdruckkammern verbunden sind

39. Hüftgelenkimplantat gemäss Anspruch 36, welches eine hohle Gelenkkugel ( 601 ) enthält, in deren Wand eine Öffnung angebracht ist um den Durchgang der Extremität einer Befestigungsstange ( 600 ) zu erlauben, wobei die genannte Dämpfungsvorrichtung in dieser Kugel zwischen der Kugelwand und der Extremität der Befestigungsstange eingebaut ist.

40. Hüftgelenkimplantat gemäss Anspruch 39 , in welchem die genannte Dämpfungsvorrichtung ein Extremitätsteil der Befestigungsstange enthält, der so angebracht ist, dass er in eine Aushöhlung einer inneren Scheidewand der Kugel gleiten kann, wobei diese Scheidewand zwei Kammern in der Kugel definiert und wobei mindestens eine kalibrierte Öffnung zwischen den beiden genannten Kammern im Extremitätsteil vorgesehen ist.

41. Hüftgelenkimplantat gemäss Anspruch 39, in welchem die Dämpfungsvorrichtung ein Extremitätsteil (603) der Befestigungsstange enthält, der zwischen zwei Dämpfungsorganen ( 608 ) liegt, von denen jedes mindestens zwei Niederdruckkammern ( 609, 610 ) besitzt, deren Wände aus elastischem Material bestehen, wobei diese Kammern durch mindestens ein mit Flüssigkeit gefülltes kalibriertes Rohr ( 613 ) verbunden sind und bei relativen Bewegungen zwischen der Kugel und dem Endstück einer Differenzialdruckveränderung ausgesetzt werden.

42. Implantat gemäss einer der Ansprüche 1 bis 41 , in welchem die Dämpfungsvorrichtung mindestens zwei Kammern enthält, die ihrerseits durch mindestens ein kalibriertes Rohr ( 313, 425 ) verbunden und mit Flüssigkeit gefüllt sind und so angelegt sind, dass sie bei relativen Bewegung der Bestandteile einer Differenzialdruckänderung ausgesetzt werden, wobei der Durchschnitt des kalibrierten Rohres von dem Druck in einer Hochdruckkammer ( 314, 434 )abhängig ist.

43. Implantatpaar gemäss einer der Ansprüche 1 bis 42, welche besonders einer Arthrodese der Wirbelsäule angepasst ist wobei jedes Implantat mechanisch in Reihenfolge an eine an sich bekannte Verbindungsstange (704, 704') angeschlossen ist.

44. Implantatpaar gemäss Anspruch 43, in welchem jedes Implantat ein Mittel zur Einstellung der Distanz ( 702 ) zwischen den durch ihn verbundenen Bestandteilen enthält.

45. Implantatpaar gemäss Anspruch 44, in welchem diese Einstellungsmittel für jedes Implantat ein mit Flüssigkeit beaufschlagbares aufblasbares Organ wie beispielsweise einen Balg, und Mittel zum Anschluss ( 705, 706, 709 )dieses Balges an eine Hochdruckflüssigkeitsquelle besitzt.

46. Implantatpaar gemäss Anspruch 45, in welcher diese Druckquelle ein Hochdruckflüssigkeitsbehälter ( 708 ) ist.

47. Implantatpaar gemäss Anspruch 46, in welchem dieser Hochdruckbehälter beiden Implantaten gemeinsam ist , jedes aufdehnbares Organ auch mit einem Niederdruckbehälter (710) verbunden ist, und eine Selbstautladungszelle ( 703 ) mechanisc in Reihenfolge an jeder Stange angeschlossen ist, wobei jede Selbstaufladungszelle durch Rückschlagventile mit den Niederdruck- beziehungsweise Hochdruckbehältern verbunden ist,und wobei eines der Ventile (715) den Abfluss der Flüssigkeit aus dem Niederdruckbehälter zu der Selbstaufladungszelle und das andere (714) den Abfluss der Flüssigkeit aus der Selbstaufladungszelle zu dem Hochdruckbehälter erlaubt.

48. Implantatpaar gemäss Anspruch 47, aus Implantaten gemäss Anspruch 2 gebildet, in welchem die Hochdruckskammern der Implantate durch ein steuerbares Ventil mit dem Hochdrucksbehälter verbunden sind

49. Implantat gemäss einen der Ansprüche 1 bis 42, welches Sensoren für physikalische Grösse ( 732 ), besonders Druck enthält, welche elektrisch versorgt sind und einer nicht invasiven ausserkörperlichen Steuerung unterstellt sind und so geschaltet sind, dass sie ihre Messungen an externe Anzeiger übersenden.

50. Implantat gemäss Anspruch 49, welcher für seine Einstellung wirksame Antreiber ( 733 ) enthält, welche ebenfalls elektrisch betrieben und nicht invasiv durch ausserkörperlichen Steurung eingestellt werden.

51. Implantat gemäss einer der vorangegangenen Ansprüche, welches Vorrichtungen (319) für Selbstanpassungen an die Bewegungen des Implantatsträger enthalten.

## Claims

1. A skeletal implant for connecting at least two elements (4, 5) of the skeleton, said implant being embodied in at least two parts (7, 8) each of which is capable of being connected to one of said elements, **characterized in that** it comprises between said two parts at least one shock-absorbing device (9) with an adjustable damping coefficient and/or means for adjusting the distance between said two parts, said shock-absorbing device and/or said adjusting means comprising one or several variable volume elements containing an hydraulic fluid, said implant further comprising a fluid refill means being responsive to corporal movements in order to send pressurized fluid to said elements.

2. An implant according to claim 1, **characterized in that** said refill means (703) are serially located between said two parts (704,704) so as to be actuated by the corporal displacements of said parts.

3. An implant according to either of claims 1 and 2 comprising means (241) for limiting the travel of the shock-absorbing device.

4. An implant according to claim 3, in which said means for limiting the travel are adjustable.

5. An implant according to any of claims 1 through 4, embodied in the form of an elongated element such as a screw, comprising two end parts (105,106) connected by a flexible middle part (107), the middle part comprising two chambers (111,112) filled with hydraulic fluid, disposed on either side of a neutral axis and designed such that one increases in volume while the other decreases in volume when the implant flexes, said chambers being connected by at least one calibrated conduit (113, 114).

6. An implant according to claim 5, in which said end parts are connected by an elastic wall (110) delimiting said chambers with a peripheral bellows.

7. An implant according to either of claims 5 or 6, in which said calibrated conduit is embodied in the form of borings in at least one of the end parts.

8. An implant according to any of claims 5 through 7, comprising a valve (116) on said calibrated conduit

9. An implant according to any of claims 1 through 4, in which the shock-absorbing device comprises at least one chamber (220) formed by two semi-chambers joined by a bellows (222), each of the semi-chambers being connected to one of the parts of the implant, said chamber being filled with a hydraulic fluid, and at least one calibrated opening (229) being provided in a wall of said chamber for connecting said chamber with another chamber (221).

10. An implant according to claim 9, in which said semi-chambers are in the shape of cupels (224,225) whose openings face one another.

11. An implant according to either of claims 9 and 10, comprising a removable, elongated stop element (243) engaged in a fold of said bellows so as to prevent its collapse,

12. An implant according to any of claims 9 through 11, comprising a stop ring (241) screwed onto one of said semi-chambers and designed to cooperate with a shoulder (242) of the other semi-chamber so as to prevent these two semi-chambers from moving toward one another beyond a certain limit.

13. An implant according to any of claims 9 through 12, in which said other chamber is housed in a support skirt (230) mounted on one of said semi-chambers around the calibrated opening, opposite the other semi-chamber.

14. An implant according to any of claims 9 through 13, in which said other chamber is also formed of two semi-chambers joined by a bellows (223).

15. An implant according to any of claims 1 through 4, also comprising elastic means between said two parts.

16. An implant according to claim 15, in which the coefficient of elasticity is adjustable.

17. An implant according to either of claims 15 and 16, comprising two chambers filled with hydraulic fluid and joined by a calibrated opening, at least one of these chambers containing a compression ampulla (411) at ambient pressure with elastic walls.

18. An implant according to claim 17, in which the wall of said ampulla has a progressive elasticity, and one of said chambers is capable of being connected to a source (415, 416) of fluid under pressure,

19. An implant according to any of claims 5 through 18, comprising means (430-435) for adjusting the cross-section of said calibrated opening.

20. An implant according to claim 19, in which said meas for adjusting the cross-section of the calibrated opening comprise a hydraulically controlled needle valve.

21. An implant according to any of claims 1 through 4, in which the shock-absorbing device comprises at least two low-pressure chambers (308, 309) whose walls are made of elastic material, said chambers being connected by at least one calibrated conduit (313) and filled with hydraulic fluid, and designed to undergo a differential pressure variation during a relative movement of said elements.

22. An implant according to claim 21, in which one of the low-pressure chambers has a wall (312) of relatively low elasticity relative to the elasticity of the walls (311) of the other chamber.

23. An implant according to either of claims 21 and 22, in which the cross-section of said calibrated conduit is determined by the prevailing pressure in a high-pressure chamber (314) which is capable of compressing this conduit

24. An implant according to claim 23, in which a high-pressure chamber and a low-pressure chamber are connected, in the low-pressure to high-pressure direction, by an anti return valve (310).

25. An implant according to claim 24, in which said anti-return valve comprises a flexible tube (511) connected to the low-pressure chamber, one free end of which is engaged in a free end of a tube (510) connected to the high-pressure chamber.

26. An implant according to either of claims 24 and 25, in which said chambers are also connected by a pressure control valve (320) mounted in parallel with the anti-return valve.

27. An implant according to the combination of claim 22 and any of claims 25 through 27, in which said anti-return valve is disposed between the high-rigidity, low-pressure chamber and the high-pressure chamber.

28. An implant according to any of claims 21 through 27, comprising a first annular low-pressure chamber (308) and a second revolving chamber (309) at the center of the first chamber, said calibrated conduits being formed radially inside the wall (310) separating the two chambers.

29. An implant according to claim 28, in which the outer wall of the first low-pressure chamber is relatively thin, and the wall separating the two low-pressure chambers is relatively thick.

30. An implant according to either of claims 28 and 29, comprising at least one annular high-pressure chamber (314) formed within the thickness of the wall separating the two low pressure chambers, and designed to compress said calibrated conduits.

31. An implant according to any of claims 21 through 29, in which said conduit is at least partially embodied in the form of a tube made of elastic material surrounded by a substantially more rigid tube, said tubes being joined into rings at their ends, the volume compressed between the two tubes forming a high-pressure chamber.

32. An implant according to any of claims 23 through 27, substantially in the form of a disk, comprising a plurality of low-pressure chambers (325) in the form of sectors, connected by said calibrated conduits (326), which themselves alternate within the thickness of the disk with said high-pressure chambers (327).

33. An implant according to any of claims 1 through 32, comprising means (317) for adjusting the distance between said two elements which it connects.

34. An implant according to claim 33, in which said adjusting means comprise a bellows (315) designed to receive a hydraulic fluid, and means for connecting said bellows to a source of fluid under pressure,

35. An implant according to any of claims 24 through 27, the low-pressure chamber of each of the implants being connected by said anti-return valve to the high-pressure chamber of the other implant

36. An implant according to any of claims 1 through 34, in which each of said parts (402,403) is articulated to the other.

37. An implant according to claim 36, in which said parts have complementary surfaces (404, 405) which rest against one another, forming a ball joint.

38. An implant according to claim 36, comprising apivot (407) integral with one of said parts and housed in a space formed between a plurality of low-pressure chambers (502) in the form of sectors integral with the other part of the implant and connected by said calibrated conduits, which themselves alternate with high-pressure chambers,

39. A coxofemoral implant according to claim 36, comprising an articulating hollow sphere (601), whose wall is open so as to allow the passage of the end of a connecting piece (600), said shock-absorbing device being disposed inside said sphere between the wall of the latter and the end of the connecting piece.

40. A coxofemoral implant according to claim 39, in which said shock-absorbing device comprises an end element of the connecting piece designed to slide through an opening of a partition inside the sphere, said partition delimiting two chambers in the sphere, and at least one calibrated opening being formed in said end element between said two chambers.

41. A coxofemoral implant according to claim 39, in which said shock-absorbing device comprises an end element (603) of the connecting piece disposed between two shock-absorbing elements (608), each of which comprises at least two low-pressure chambers (609, 610) whose walls are made of elastic material, said chambers being connected by at least one calibrated conduit (613) and filled with hydraulic fluid, and designed to undergo a differential pressure variation during a relative movement of the sphere and the connecting piece.

42. An implant according to any of claims 1 through 41, in which the shock-absorbing device comprises at least two chambers, said chambers being connected by at least one calibrated conduit (313; 425) and filled with hydraulic fluid, and designed to undergo a differential pressure variation during a relative movement of said elements, the cross-section of said calibrated conduit being determined by the prevailing pressure in a high-pressure chamber (314; 434),

43. A pair of implants according to any of claims 1 through 42, particularly used within the scope of an arthrodesis of the vertebral column, each of the implants being mechanically connected in series to a connecting pin (704,704') of a known type.

44. A pair of implants according to claim 43, in which each of the implants comprises means (702) for adjusting the distance between said elements which it connects.

45. A pair of implants according to claim 44, in which said adjusting means comprise, for each implant, an expandable element such as a bellows, designed to receive a hydraulic fluid, and means (705, 706; 709) for connecting said bellows to a source of fluid under pressure.

46. A pair of implants according to claim 45, in which each source of fluid under pressure is a reservoir (708) of high-pressure fluid

47. A pair of implants according to claim 46, in which said high-pressure reservoir is common to both implants, each expandable element is also connected to a low-pressure reservoir (710), and an expandable refill cell (703) is mechanically connected in series to each pin, each refill cell being connected to the high-pressure and low-pressure reservoirs by two anti-return valves, one (715) allowing a flow of fluid from the low-pressure reservoir to the refill cell, and the other (714) allowing a flow of fluid from the refill cell to the high-pressure reservoir,

48. A pair of implants according to claim 47, formed of implants according to claim 2, and in which the high-pressure chambers of the implants are connected to the high-pressure reservoir by a controllable valve.

49. A skeletal implant according to any of claims 1 through 42, comprising sensors of physical quantities (732), including pressure, supplied with electricity and controlled from outside the body in a non-invasive way, and designed to transmit their information to display means (727).

50. A skeletal implant according to claim 49, comprising adjustment controls (733), also supplied with electricity and controlled from outside the body in a rion-invasive way.

51. An implant according to any of the preceding claims, comprising means (319) for automatically adapting to the movements of the wearer of the implants.
